(19) 

**Europäisches Patentamt**
**European Patent Office**
**Office européen des brevets**

(11) **EP 4 389 772 A1**

(12) 

# EUROPEAN PATENT APPLICATION
published in accordance with Art. 153(4) EPC

(43) Date of publication:
**26.06.2024 Bulletin 2024/26**

(21) Application number: **22858839.8**

(22) Date of filing: **17.08.2022**

(51) International Patent Classification (IPC):
*C07K 19/00* (2006.01)      *C12N 9/96* (2006.01)
*A61K 47/66* (2017.01)      *A61K 47/68* (2017.01)
*A61K 38/46* (2006.01)      *A61K 39/395* (2006.01)
*A61K 48/00* (2006.01)      *C12N 9/14* (2006.01)

(52) Cooperative Patent Classification (CPC):
**A61K 38/46; A61K 39/395; A61K 47/66;**
**A61K 47/68; A61K 48/00; C07K 19/00; C12N 9/96;**
**C12N 9/14**

(86) International application number:
**PCT/RU2022/050251**

(87) International publication number:
**WO 2023/022629 (23.02.2023 Gazette 2023/08)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **18.08.2021 RU 2021124495**

(71) Applicant: **Joint-Stock Company "Generium"**
**Petushinskiy rayon, pos. Volginskiy, 601125 (RU)**

(72) Inventors:
 • **SHUKUROV, Rakhim Rakhmankulyyevich**
  **Borovichi, 174411 (RU)**

 • **KHAMITOV, Ravil Avgatovich**
  **Moscow, 125466 (RU)**
 • **SHUSTER, Aleksandr Mikhailovich**
  **Moscow, 115184 (RU)**
 • **RESHETNIK, Elizaveta Vyacheslavovna**
  **Republic of Tatarstan, Kazan, 420133 (RU)**

(74) Representative: **Studio Torta S.p.A.**
**Via Viotti, 9**
**10121 Torino (IT)**

Remarks:
The complete document including Reference
Table(s) and the Sequence Listing(s) can be
downloaded from the EPO website

(54) **TARGETED DELIVERY OF THERAPEUTIC ENZYMES**

(57)    The invention relates to the field of biotechnology, and more particularly to the delivery (transport) of therapeutic enzymes, which is applicable in medicine. The present invention relates to a compound containing a therapeutic enzyme and a transport element that are coupled to one another directly or by a linker, the transport element being a Fab fragment of immunoglobulin IgG specific to an insulin receptor epitope, and to the use of said compound to produce a pharmaceutical composition for treating diseases, as well as to the use of said compound for the treatment and prophylaxis of diseases, in particular lysosomal storage diseases, inter alia, for the treatment and prophylaxis of the enzyme deficiency characteristic of the respective lysosomal storage disease, such as mucopolysaccharidosis, in particular mucopolysaccharidosis types I and II.

EP 4 389 772 A1

**Description**

**Technical field**

The invention relates to the field of biotechnology, and more particularly to the delivery (transport) of therapeutic enzymes, which is applicable in medicine. The present invention relates to a compound containing a therapeutic enzyme and a transport element that are coupled to one another directly or by a linker, the transport element being a Fab fragment of immunoglobulin IgG specific to an insulin receptor epitope, and to the use of said compound to produce a pharmaceutical composition for treating diseases, as well as to the use of said compound for the treatment and prophylaxis of diseases, in particular lysosomal storage diseases, inter alia, for the treatment and prophylaxis of the enzyme deficiency characteristic of the respective lysosomal storage disease, such as mucopolysaccharidosis, in particular mucopolysaccharidosis types I and II.

**Background art**

The use of enzymes in therapy has been known in the art for a long time. Contemporary medicine is widely using therapeutic enzymes in various branches of medicine due to their high activity and specificity. Currently, the following directions of enzyme therapy have emerged (Kazanskaya N. F. et al., 1984): 1) elimination of enzyme deficiency in order to compensate for congenital or acquired functional deficiency; 2) removal of non-viable, denatured structures, cellular and tissue fragments; 3) lysis of blood clots; 4) complex therapy of malignant neoplasms; 5) detoxification of the body.

The use of therapeutic enzymes to eliminate enzyme deficiency in order to compensate for congenital or acquired functional deficiency has been practiced for a long time. Treatment of congenital enzyme deficiencies, of which more than 150 have been described, is an important problem in replacement therapy. Hereditary diseases such as glycogenosis, lipidosis, mucopolysaccharidosis and other lysosomal storage diseases are predominantly treated by intravenous administration of recombinant analogues of the corresponding native enzymes.

Lysosomal storage diseases are a group of rare (orphan) hereditary metabolic diseases caused by the absence or deficiency of lysosomal enzymes involved in the breakdown of complex molecules.

Currently (Novikov P.V., 2014) the following groups of lysosomal storage diseases are distinguished: 1) mucopolysaccharidoses; 2) lipidoses (sphingolipidoses - GM1- and GM2-gangliosidoses, Gaucher disease, galactosialidosis, Farber granulomatosis, leukodystrophies, Niemann-Pick disease types A and B, etc.); 3) mucolipidoses, 4) glycoproteinoses (fucosidosis, sialidosis, mannosidosis, glycogen storage disease type II - Pompe disease, Danon disease, etc.); 5) neuronal ceroid lipofuscinoses; 6) other storage diseases (Niemann-Pick disease type C, Wolman disease, cholesterol storage disease, cystinosis, Sal's disease, pycnodysostosis, etc.).

Mucopolysaccharidoses (MPS) are a group that unites nine (I - IX, with intermediate forms - 14) metabolic diseases caused by more than 40 genetic disorders leading to the absence or functional deficiency of a number of lysosomal enzymes involved in the hydrolytic breakdown of glycosaminoglycans (mucopolysaccharides). Glycosaminoglycans are oligosaccharides that participate in the formation of bones, cartilage, ligaments, cornea, skin, connective tissue and synovial fluid (Burrow T. A. et al, 2013).

In patients suffering from mucopolysaccharidosis, there is a deficiency or absence of at least one of the 11 enzymes of glycosaminoglycan catabolism, which over time leads to the gradual accumulation of these carbohydrates in cells and body fluids, incl. connective tissue. As a result, the permanent accumulation of mucopolysaccharides leads to cell damage, dysfunction of tissues and organs, most often manifested in hypertension-hydrocephalic syndrome, hepatosplenomegaly, cardiovascular failure, osteoarticular complications, functional disorders of the central nervous system (CNS), which includes severe cognitive disorders and dementia (Table 1) (Burrow T. A. et al, 2013).

Table 1. Characteristics of various MPS syndromes

| Syndrome | Enzyme defect | Defective chromosome, mode of inheritance | Clinical picture, laboratory data | Neurological component |
|---|---|---|---|---|
| Hurler (MPS-I-H) | $\alpha$-L-iduronidase | Chromosome 4, autosomal recessive | Children die at the age of 6-10 years from cardiac and pulmonary decompensation. Increased levels of heparan and dermatan sulfates in urine (Connock et al. 2006) | Present |

(continued)

| Syndrome | Enzyme defect | Defective chromosome, mode of inheritance | Clinical picture, laboratory data | Neurological component |
|---|---|---|---|---|
| Scheie (MPS-I-S) | $\alpha$-L-iduronidase (partial) | Chromosome 4, autosomal recessive | Mild subtype, normal intelligence, normal stature. Patients live up to 30 years. Elivated levels of heparan and dermatan sulfates in urine (Connock et al. 2006, Pastores et al. 2007). | Absent |
| Hunter (MPS II) | Iduronate-2-sulfatase | X-linked, recessive type | Boys suffer. Mental development is reduced. Patients live up to 30-40 years. There is no corneal opacity. There is no dorsolumbar hump. Characterized by progressive deafness and hirsutism. Increased levels of heparan and dermatan sulfates in urine (Wraith et al. 2008, da Silva et al. 2016, Burrow and Leslie 2008). | Present |
| Sanfilippo (MPS-III) | Type A - sulfamidase. Type B - b-N-acetylglucosaminidase Type C - acetyl-CoA-glucosamine-N-acetyltransferase Type D - a-N-acetylglucosamine-6-phosphatase | Chromosomes 12 and 17, autosomal recessive | Severe retardation of intelligence, macrocephaly, very aggressive behavior. Relatively weak skeletal changes. Visceromegaly appears at school age. Clinically, the types cannot be differentiated. Heparan sulfate in urine - (Valstar et al. 2008). | Present |
| Morquio (MPS-IV) | N-acetylgalactosamine-6-sulfatase (type A) and b-galactosidase (type B) | Chromosomes 3 and 16, autosomal recessive | Normal intelligence. Severe skeletal deformation. Corneal opacity. Less degree of central nervous system damage. The first clinical signs appear at the age of 2-3 years. Patients die at the age of 20-35 years. Keratan sulfate in urine (Northover, Cowie and Wraith 1996). | Absent |
| Maroteaux-Lamy (MPS-VI) | N-acetylgalactosamine-4-sulfatase | Chromosome 5, autosomal recessive | Slight growth retardation, pronounced hump, normal intelligence, mild facial abnormalities. Dermatan sulfate in urine (Garrido et al. 2008). | Absent |
| Sly (MPS-VII) | b- glucoronidase | Chromosome 7, autosomal recessive | Variable mental development, pronounced hump, hepatosplenomegaly, mild facial abnormalities. Dermatan sulfate in urine. | Present |

Mucopolysaccharidosis type I is caused by a deficiency of the lysosomal enzyme, $\alpha$-L-iduronidase. This deficiency leads to the accumulation of mucopolysaccharides, especially dermatan sulfate, in tissues and organs. The accumulation of excess dermatan sulfate leads to the gradual development of numerous morphological abnormalities in tissues and organs. Mucopolysaccharidosis type I is characterized by an autosomal recessive mode of inheritance.

To date, two effective methods of treating MPS type I have been developed: hematopoietic stem cell transplantation (HSCT) and enzyme replacement therapy (ERT).

HSCT is used to treat only severe forms of MPS type I - Hurler syndrome, which makes it possible to correct the deficiency of the enzyme α-L-iduronidase and, in turn, leads to a significant improvement in the patient's condition, although some severe complications of the disease do not completely regress. HSCT should be performed as early as possible, before the onset of severe neurological disorders. Despite the improvement in the patient's condition, HSCT is accompanied by a high risk of serious post-transplant complications and is a complex, multi-stage, high-cost procedure.

ERT is safe, well tolerated by patients, does not cause severe adverse events and leads to the decomposition of the non-hydrolyzed substrate. Possible rare reactions to the administration of the drug are caused by the formation of antibodies against the injected protein, but they are not permanent and, as a rule, are quickly relieved by standard medications. The principle of ERT is based on restoring the level of enzymatic activity sufficient to hydrolyze accumulated substrates and prevent their further accumulation.

The drug Aldurazyme (INN: laronidase) (Genzyme, USA) is intended for ERT in patients with three clinical variants of type I mucopolysaccharidosis (IH, IH/S and IS types or Hurler, Hurler-Scheie and Scheie syndromes), as well as children with Hurler syndrome waiting for HSCT (during the search for a relative/ non-relative donor) and during 3 to 6 months after HSCT until the child's condition is stabilized; Along with this, Aldurazym is indicated for patients suffering from Hurler syndrome after HSCT in cases where the level of the donor enzyme α-L-iduronidase remains low.

Laronidase (Bayomarin Pharmaceutical Inc., USA) is a recombinant form of human α-L-iduronidase, produced using recombinant DNA technology using Chinese hamster ovary cell culture. It has been established that after intravenous administration, laronidase quickly leaves the systemic circulation and is absorbed by cells through mannose-6-phosphate receptors (M6PR), entering lysosomes. The drug is administered intravenously (at a dose of 100 units/kg body weight), for 3-4 hours, once a week.

Laronidase is a recombinant protein of 628 amino acid residues with a molecular weight of approximately 83 kDa. Its molecule contains 6 sections that bind through the amino group to oligosaccharides of different structures, including those containing mannose-6-phosphate (M6P) residues (Rodney J.Y. Ho, Biotechnology and biopharmaceuticals: transforming proteins and genes into drugs. - Wiley-Blackwell, 2013 , p. 380). It has been established that the laronidase molecule does not penetrate the blood-brain barrier (the physiological barrier between the peripheral circulation and the brain, which is formed by the tight junctions of the plasma membranes of the endothelial cells of the brain capillaries, creating a tight barrier that limits the transfer of molecules into the brain, even very small molecules; hereinafter also indicated as BBB) and, thus, does not slow down or prevent the development of disorders of the central nervous system (CNS) characteristic of mucopolysaccharidosis type I (Pastores G.M. et al, 2007).

Mucopolysaccharidosis type II (MPS-II, Hunter syndrome), unlike all other forms of mucopolysaccharidosis that have an autosomal recessive mode of inheritance, is the only form of mucopolysaccharidosis with an X-linked recessive mode of inheritance. MPS II is a heterogeneous according to clinical manifestations, progressive pathology of lysosomal accumulation, resulting from deficiency of the enzyme iduronate-2-sulfatase (idursulfase). This enzyme is normally involved in the degradation of mucopolysaccharides dermatan sulfate and heparan sulfate due to the hydrolytic removal of the O-linked sulfate group. Accordingly, in the case of MPS II, the main pathogenetic mechanism is associated with the progressive accumulation of dermatan and heparan sulfates in lysosomes

The most common clinical symptoms of MPS-II are mental retardation, enlarged tongue, characteristic pathologies of the facial skeleton, alopecia, dental anomalies, restrictive lung disease, hepatosplenomegaly, heart valve pathology, osteoarticular pathologies, severe short stature. Also, progressive neurological disorders are often observed, accompanied by hydrocephalus and increased intracranial pressure. Death usually occurs during the second to third decade of life, most often due to respiratory and/or heart failure. It is important to emphasize that the disease occurs with the involvement of the nervous system in the pathological process, including cognitive decline.

Today, there are two main methods of treating MPS II - ERT and symptomatic therapy (includes the use of hepatoprotectors, cardiovascular and anti-inflammatory drugs, vitamins and drugs that improve antioxidant protection). HSCT for the treatment of MPS II, unlike mucopolysaccharidosis type I, is not effective.

Elaprase (INN: idursulfase) (Shayer, USA) is a medical product representing recombinant human iduronate-2-sulfatase. This enzyme is responsible for the hydrolysis of C2-sulfate ester bonds of iduronic acid residues, which are part of the glycosaminoglycans (mucopolysaccharides) of dermatan sulfate and heparan sulfate (Burrow T. A. et al., 2013). The normal dosing regimen for Elaprase is 0.5 mg/kg body weight, once a week; administration is carried out by intravenous infusion during 3 hours (infusion time can be gradually reduced to 1 hour).

Idursulfase contains two disulfide bonds and eight N-linked glycosylation sites occupied by complex high-mannose oligosaccharides. The presence of M6P residues in the oligosaccharide chains allows the recombinant enzyme to bind to M6P receptors on the surface of target cells, which leads to the internalization of this enzyme into cells and its internalization into lysosomes, where it ensures the degradation of lysosomal mucopolysaccharides (Muenzer J., et al., Genet Med 2006 Aug;8(8):465 - 473).

When performing ERT, the life expectancy of patients with Hunter syndrome increases several times. Despite this, Elaprase also does not penetrate the blood-brain barrier; as a result, when performing ERT with Elaprase, patients die at the age of 20 from neurodegenerative consequences, and in the second decade of life, patients receiving Elaprase, as a rule, experience great learning difficulties and need helpers even in everyday life (da Silva E. M. et al., 2016; Wraith J.E. et al., 2008).

Thus, a common disadvantage of all currently existing drugs used for ERT of mucopolysaccharidosis types I and II is their inability to penetrate the blood-brain barrier into the central nervous system, which limits the effectiveness of the use of these drugs in patients with nervous system damage associated with mucopolysaccharidosis, including mucopolysaccharidosis types I and II

For many lysosomal storage diseases, there is a need for improved internalization of the enzyme into the cells of certain peripheral tissues (diaphragm muscles in Pompe disease, liver and spleen in Hunter disease, kidneys in Fabry disease, etc.) (Hawkes C. et al., 2004)

Thus, there is an urgent need to develop a therapeutic compound that could be used to treat a lysosomal storage disease, such as MPS type I or MPS type II, and which would retain the functional properties of the corresponding therapeutic enzyme, while demonstrating high activity and improved ability to be transported to lysosomes of tissue cells of various organs, including lysosomes of nervous tissue cells, and would make it possible to achieve a significant improvement in the quality and life expectancy of patients with MPS types I and II.

**Advantages of invention**

The above objects are successfully solved in the present invention, which relates to a compound intended for the treatment of lysosomal storage disease, containing a therapeutic enzyme and a transport element coupled to each other directly or by a linker, wherein the specified transport element is a Fab fragment of an IgG immunoglobulin, specific to an insulin receptor epitope. The invention is based on the unexpected discovery that a transport element, which is a Fab fragment of an IgG immunoglobulin specific to an insulin receptor epitope, coupled directly or by a linker to a therapeutic enzyme, unexpectedly leads to an improvement in the ability of the enzyme to be transported to the lysosomes of various organs and tissues, including to the lysosomes of nervous tissue cells, and at the same time the enzymatic activity of the compound remains at a high level. Thanks to this unexpected effect, progress is being made in the field of enzyme replacement therapy for lysosomal storage diseases such as mucopolysaccharidosis types I and II, and increasing the duration and improving the quality of life of subjects suffering from lysosomal storage diseases and requiring therapy. The compound of the present invention also provides an expansion of the arsenal of agents used for the treatment and prevention of diseases, in particular lysosomal storage diseases such as mucopolysaccharidosis, in particular mucopolysaccharidosis types I and II.

In addition, an unexpected increase in the enzymatic activity of a compound containing a transport element, which is a Fab fragment of an IgG immunoglobulin specific for an insulin receptor epitope, coupled directly or by a linker to a therapeutic enzyme, was found, compared with a therapeutic enzyme without a transport element; and also that the introduction of a compound containing a transport element, which is a Fab fragment of an IgG immunoglobulin specific for an insulin receptor epitope, coupled directly or by a linker to a therapeutic enzyme provides a higher degree of replacement of the activity of the therapeutic enzyme in the human brain in comparison with compounds, containing a Mab fragment.

**Summary of Invention**

This Summary of Invention presents a brief description of the present invention in order to briefly outline the subject matter and essence of the present invention. The Summary of Invention is provided with due appreciation that it should not be used to interpret or limit the scope or content of the claims.

The first subject matter of the present invention is a compound intended for the treatment of lysosomal storage disease, containing a therapeutic enzyme and a transport element coupled to each other directly or by a linker, wherein the transport element is a Fab fragment of immunoglobulin IgG specific for an insulin receptor epitope.

In a specific embodiment, the present invention provides a compound for the treatment of a lysosomal storage disease, comprising a therapeutic enzyme and a transport element coupled to each other directly or by a linker, wherein the transport element is a Fab fragment of immunoglobulin IgG specific for an insulin receptor epitope, and is capable of transporting this enzyme through the blood-brain barrier.

In a specific embodiment, the present invention provides a compound for the treatment of a lysosomal storage disease comprising a therapeutic enzyme and a transport element, wherein the transport element is a Fab fragment of immunoglobulin IgG specific for an insulin receptor epitope and is capable of transporting said enzyme through the blood-brain barrier, wherein the therapeutic enzyme and the transport element are coupled to each other by a linker. The linker may be a peptide linker containing one or more amino acids. The linker may be a peptide linker containing one or more amino acids selected from glycine, serine and leucine.

In a specific embodiment, the present invention provides a compound for the treatment of a lysosomal storage disease, comprising a therapeutic enzyme and a transport element coupled to each other directly or by a linker, wherein the transport element is a Fab fragment of immunoglobulin IgG, wherein the immunoglobulin IgG is IgG1 , IgG2 or IgG4.

In a specific embodiment, the present invention provides a compound for the treatment of a lysosomal storage disease, comprising a therapeutic enzyme and a transport element coupled to each other directly or by a linker, wherein the transport element is a Fab fragment of immunoglobulin IgG, wherein the immunoglobulin IgG is IgG1 .

In a specific embodiment, the present invention provides a compound for the treatment of a lysosomal storage disease, containing a therapeutic enzyme and a transport element coupled to each other directly or by a linker, wherein the transport element is a Fab fragment of immunoglobulin IgG1 consisting of immunoglobulin IgG1 light chains to the insulin receptor (SEQ ID NO:2, 8, 9, 10 and 11) and the heavy chain (SEQ ID NO:3). In a particular embodiment, the subject matter of the present invention is a compound where the transport element is a Fab fragment of immunoglobulin IgG1, consisting of a light chain of an immunoglobulin IgG1 to insulin receptor SEQ ID NO:2 and a heavy chain SEQ ID NO:3.

In a specific embodiment, the present invention is a compound for the treatment of a lysosomal storage disease, containing a therapeutic enzyme and a transport element coupled to each other directly or by a linker, wherein the therapeutic enzyme is used for the treatment of a lysosomal storage disease, including the treatment of an enzyme deficiency characteristic of this lysosomal storage disease.

In a specific embodiment, the present invention provides a compound for the treatment of a lysosomal storage disease, comprising a therapeutic enzyme and a transport element coupled to each other directly or by a linker, wherein the therapeutic enzyme is used to treat a lysosomal storage disease with a neurological component, including, for the treatment of enzyme deficiency characteristic of this lysosomal storage disease.

In a specific embodiment, the present invention provides a compound for the treatment of a lysosomal storage disease, comprising a therapeutic enzyme and a transport element coupled to each other directly or by a linker, wherein the therapeutic enzyme for a lysosomal storage disease with a neurological component is selected from the group consisting of iduronate -2-sulfatases and $\alpha$-L-iduronidases.

In a specific embodiment, the present invention provides a compound for the treatment of a lysosomal storage disease, comprising a therapeutic enzyme and a transport element coupled to each other directly or by a linker, wherein the therapeutic enzyme for a lysosomal storage disease with a neurological component is selected from the group consisting of iduronate-2-sulfatase, a fragment of iduronate-2-sulfatase having iduronate-2-sulfatase activity, or an iduronate-2-sulfatase analogue.

In a specific embodiment, the present invention provides a compound for the treatment of a lysosomal storage disease, comprising a therapeutic enzyme and a transport element coupled to each other directly or by a linker, wherein the therapeutic enzyme for a lysosomal storage disease with a neurological component is selected from the group consisting of $\alpha$ -L-iduronidase, a fragment of $\alpha$-L-iduronidase having $\alpha$-L-iduronidase activity, or an $\alpha$-L-iduronidase analogue.

In a specific embodiment, the present invention provides a compound for the treatment of a lysosomal storage disease comprising a therapeutic enzyme and a transport element coupled to each other directly or by a linker, wherein said linker is a peptide linker containing one or more amino acids.

In a specific embodiment, the present invention provides a compound for the treatment of a lysosomal storage disease comprising a therapeutic enzyme and a transport element coupled to each other directly or by a linker, wherein said linker is a peptide linker containing one or more amino acids selected from glycine, serine and leucine.

In a specific embodiment, the present invention provides a compound for the treatment of a lysosomal storage disease comprising a therapeutic enzyme and a transport element coupled to each other directly or by a linker, wherein said transport element is capable of transporting said enzyme to lysosomes of tissue cells.

In a specific embodiment, the present invention provides a compound for the treatment of a lysosomal storage disease comprising a therapeutic enzyme and a transport element coupled to each other directly or by a linker, wherein said transport element is capable of transporting said enzyme to lysosomes of neural tissue cells.

Another subject matter of the invention is a compound for the treatment or prevention of an enzyme deficiency (iduronate-2-sulfatase deficiency) in a subject having mucopolysaccharidosis type II, represented by the first amino acid sequence selected from SEQ ID NO: 2, 8, 9, 10 or 11, and a second amino acid sequence selected from SEQ ID NO: 4, 5, 12, 13, 14 or 15. In a particular embodiment, the present invention provides a compound for the treatment or prevention of an enzyme deficiency (iduronate-2-sulfatase deficiency) in a subject having mucopolysaccharidosis type II, represented by the amino acid sequences of SEQ ID NO:2 and SEQ ID NO:4.

Another subject matter of the invention is a compound for the treatment or prevention of enzyme deficiency (iduronate-2-sulfatase deficiency) in a subject having mucopolysaccharidosis type II with a neurological component, represented by the amino acid sequences SEQ ID NO:2 and SEQ ID NO:4.

Another subject matter of the invention is a compound for the treatment or prevention of enzyme deficiency (iduronate-2-sulfatase deficiency) in a subject having mucopolysaccharidosis type II with a neurological component, represented by the amino acid sequences SEQ ID NO:2 and SEQ ID NO:5.

Another subject matter of the invention is a compound for the treatment or prevention of enzyme deficiency ($\alpha$-L-iduronidase deficiency) in a subject having mucopolysaccharidosis type I, represented by the amino acid sequences SEQ ID NO:2 and SEQ ID NO:6.

Another subject matter of the invention is a compound represented by the amino acid sequences SEQ ID NO:2 and SEQ ID NO:6, for the treatment or prevention of enzyme deficiency ($\alpha$-L-iduronidase deficiency) in a subject having mucopolysaccharidosis type I.

Another subject matter of the invention is a compound represented by the amino acid sequences SEQ ID NO:2 and SEQ ID NO:6, for the treatment or prevention of enzyme deficiency ($\alpha$-L-iduronidase deficiency) in a subject having mucopolysaccharidosis type I with a neurological component.

Another subject matter of the invention is a compound represented by the amino acid sequences SEQ ID NO:2 and SEQ ID NO:7, for the treatment or prevention of enzyme deficiency ($\alpha$-L-iduronidase deficiency) in a subject having mucopolysaccharidosis type I with a neurological component.

In addition, the present invention relates to the use of a compound containing a therapeutic enzyme and a transport element coupled to each other directly or by a linker to prepare a pharmaceutical composition containing an effective amount of this compound and a pharmaceutically acceptable carrier.

In addition, the present invention relates to the use of a compound containing a therapeutic enzyme and a transport element coupled to each other directly or by a linker for the treatment or prevention of a subject having a lysosomal storage disease, comprising administering to said subject said compound in an effective amount

In a specific embodiment, the invention relates to the use of a compound comprising a therapeutic enzyme and a transport element coupled to each other directly or via a linker for the treatment or prevention of enzyme deficiency in a subject having a lysosomal storage disease, wherein the lysosomal storage disease is mucopolysaccharidosis, wherein the compound is administered to the subject in an effective amount, preferably in conjunction with a pharmaceutically acceptable carrier.

In a specific embodiment, the invention relates to the use of a compound comprising a therapeutic enzyme and a transport element coupled to each other directly or by a linker, for the treatment or prevention of enzyme deficiency (iduronate-2-sulfatase deficiency) in a subject having a lysosomal storage disease, wherein the lysosomal storage disease is mucopolysaccharidosis type II with a neurological component, wherein the compound is administered to the subject in an effective amount, preferably in conjunction with a pharmaceutically acceptable carrier

In a specific embodiment, the invention relates to the use of a compound comprising a therapeutic enzyme and a transport element coupled to each other directly or by a linker, for the treatment or prevention of enzyme deficiency ($\alpha$-L-iduronidase deficiency) in a subject having a lysosomal storage disease, wherein the lysosomal storage disease is mucopolysaccharidosis type I with a neurological component, wherein the compound is administered to the subject in an effective amount, preferably in conjunction with a pharmaceutically acceptable carrier.

In addition, the present invention relates to the use of a compound containing a therapeutic enzyme and a transport element coupled to each other directly or by a linker, for the treatment or prevention of enzyme deficiency (iduronate-2-sulfatase deficiency) in a subject having a lysosomal storage disease, wherein lysosomal storage disease is mucopolysaccharidosis type II, wherein the compound represented by the amino acid sequences SEQ ID NO:2 and SEQ ID NO:4, for the preparation of a pharmaceutical composition containing said compound in an effective amount and a pharmaceutically acceptable carrier.

In addition, the present invention relates to the use of a compound containing a therapeutic enzyme and a transport element coupled to each other directly or by a linker, for the treatment or prevention of enzyme deficiency (iduronate-2-sulfatase deficiency) in a subject having a lysosomal storage disease, wherein lysosomal storage disease is a mucopolysaccharidosis type II, wherein the compounds represented by the amino acid sequences of SEQ ID NO:2 and SEQ ID NO:4, for the treatment or prevention of an enzyme deficiency in a subject.

In other embodiments, the approach of the present invention can be used to treat other lysosomal storage diseases, in particular, to treat other types of mucopolysaccharidosis, such as mucopolysaccharidosis type III (Sanfilippo syndrome), including mucopolysaccharidosis type IIIA, type IIIB, type IIIC and IIID type; mucopolysaccharidosis type IV (Morquio syndrome), including mucopolysaccharidosis type IVA and type IVB; mucopolysaccharidosis type VI (Maroteaux-Lamy syndrome), mucopolysaccharidosis type VII (Sly syndrome) and mucopolysaccharidosis type IX. In these cases, instead of $\alpha$-L-iduronidase or iduronate-2-sulfatase, another therapeutic enzyme is used as the appropriate therapeutic enzyme, the deficiency of which is encountered by patients with the corresponding form of mucopolysaccharidosis: heparan sulfamidase - for mucopolysaccharidosis type IIIA, N-acetylglucosaminidase - for mucopolysaccharidosis type IIIB, heparan-$\alpha$-glucosaminide-N-acetyltransferase - for mucopolysaccharidosis type IIIC, N-acetylglucosamine-6-sulfatase - for mucopolysaccharidosis type IIID, galactose-6-sulfate sulfatase - for mucopolysaccharidosis type IVA, $\beta$-galactosidase - for mucopolysaccharidosis type IVB, N- acetylgalactosamine-4-sulfatase - for mucopolysaccharidosis type VI, $\beta$-glucuronidase - for mucopolysaccharidosis type VII and hyaluronidase - for mucopolysaccharidosis type IX. The amino acid sequences of these therapeutic enzymes can be obtained using any of a number of computer programs known in the art, for example, BLAST or FASTA, etc. Both BLAST and FASTA have offline and online search capabilities (see Ausubel et al., 1999 ibid, pages 7-58 - 7-60, National Center for Biotechnology Information website at the National Institutes of Health website).

The approach proposed in the present invention, in alternative embodiments of the invention, can also be used for the treatment of lysosomal storage diseases not related to mucopolysaccharidoses, by using, instead of α-L-iduronidase or iduronate-2-sulfatase, another lysosomal enzyme, which is deficient in patients having the corresponding lysosomal storage disease. In certain non-limiting embodiments of the present invention, the proposed approach can be, in particular, applied to the treatment of disorders of amino acid metabolism, such as cystinosis; for the treatment of carbohydrate metabolism disorders, such as glycogen storage diseases, in particular Pompe disease; disorders of sphingolipid metabolism and other lipid storage diseases, in particular $GM_2$ gangliosidosis, including Sandhoff disease and Tay-Sachs disease; other gangliosidoses, in particular $GM_1$ gangliosidosis and mucolipidosis IV; other sphingolipidoses, in particular Fabry disease, Gaucher disease, Krabbe disease, Niemann-Pick disease, Farber syndrome, metachromic leukodystrophy and multiple sulfatase deficiency; lipofuscinosis of neurons, in particular, Batten, Bielschowsky-Jansky, Kufs, Spielmeyer-Vogt diseases; other lipid storage disorders, in particular Wolman's disease, as well as for the treatment of disorders of glycoprotein metabolism, including mucolipidosis II (I-cell disease), mucolipidosis III (Hurler pseudolipodystrophy), and for the treatment of defects in glycoprotein degradation, including aspartylglucosaminuria, fucosidosis, mannosidosis and sialidosis.

In these cases, instead of α-L-iduronidase or iduronate-2-sulfatase, the amino acid sequence of the corresponding enzyme, which is deficient in patients with the corresponding lysosomal storage disease, is coupled to the transport element directly or by a linker.

The most preferred embodiments of the present invention are described below. However, these preferred embodiments are provided only for purposes of illustration of the present invention and not to limit the scope of the claims.

## Brief description of the figures

The invention is illustrated by the following figures.

Fig. 1 shows a representative autoradiogram of a male cynomolgus monkey recorded 2 hours after a single intravenous administration of [$^{125}$I]-HIR-Fab-IDS at a nominal dose level of 0.0020 mg/kg body weight.
Fig. 2 shows a representative autoradiogram of a male cynomolgus monkey recorded 2 hours after a single intravenous administration of [$^{125}$I]-HIR-Mab-IDS at a nominal dose level of 0.0015 mg/kg body weight.
Fig. 3 shows a representative autoradiogram of a male cynomolgus monkey recorded 2 hours after a single intravenous administration of [$^{125}$I]-IDS (control) at a nominal dose level of 0.0010 mg/kg body weight.

## Detailed Description og the Invention

Unless otherwise stated, all technical and scientific terms used herein have the same meaning as commonly understood by an average person skilled in the art (e.g., molecular genetics, nucleic acid chemistry, protein chemistry, biochemistry, organic chemistry, immunology , microbiology, genetics, etc.).

As used herein, the term "compound" is understood in its broadest sense as at least one molecule that is a conjugate, fusion protein, protein construct, protein complex, etc.

As used herein, the term "containing" refers to a compound containing the listed elements without excluding others.

In the present specification, the terms "therapeutic enzyme" and "enzyme" are synonymous and refer to an enzyme for treating diseases resulting from the absence, deficiency, dysfunction of the enzyme and so on, wherein the subject suffering from the diseases can be treated by ERT, administration of the enzyme etc. In particular, the enzyme may be an enzyme for the treatment of diseases that may occur due to, but not limited to, absence, deficiency and dysfunction of a lysosomal enzyme.

The therapeutic enzyme that is included in the compound of the present disclosure includes any enzyme that has a therapeutic effect against a lysosomal storage disease, including, without limitation, β-glucosidase, β-galactosidase, galactose-6-sulfatase, acid ceramidase, acid sphingomyelinase, galactocerebrosidase, arylsulfatase A, β-hexosaminidase A, β-hexosaminidase B, heparin-N-sulfatase, α-D-mannosidase, β-glucuronidase, N-acetylgalactosamine-6-sulfatase, lysosomal acid lipase, α-N-acetyl-D-glucosaminidase (NAGLU), glucocerebrosidase, butyrylcholinesterase, chitinase, glutamate decarboxylase, lipase, uricase, platelet activating factor acetylhydrolase, neutral endopeptidase, myeloperoxidase, acetyl-CoA-glucosaminide-N-acetyltransferase, N-acetylglucosamine-6-sulfatase, galactosamine-6-sulfatase (GALN), hyaluronidase, α-fucosidase, β-mannosidase, α-neuraminidase (sialidase), N-acetyl-glucosamine-1-phosphotransferase, mucolipin-1, α-N-acetyl-galactosaminidase, N-aspartyl-β-glucosaminidase, LAMP-2 (lysosome-associated membrane protein 2), cystinosin, sialin, ceramidase, acid P-glucosidase, galactosylceramidase, NPC1 (Niemann-Pick disease protein type C1), cathepsin A, SUMF-1 (sulfatase-modifying factor-1), lysosomal acid lipase (LIPA) and tripeptidyl peptidase 1.

In particular, the therapeutic enzyme includes such enzymes as agalcidase, imiglucerase, galsulfase, iduronate-2-sulfatase and α-L-iduronidase. Most preferably, the therapeutic enzyme is iduronate-2-sulfatase or α-L-iduronidase.

However, the present description may also cover any therapeutic enzyme, without limitation, regardless of the type or origin of the enzyme.

In the present disclosure, the term **"iduronate-2-sulfatase",** as well as **"idursulfase", "IDS", "I2S",** means a recombinant analogue of the lysosomal enzyme iduronate-2-sulfatase, which normally hydrolyzes the O-linked sulfate group of mucopolysaccharides - dermatan and heparan sulfates. In the present invention, the term **"iduronate-2-sulfatase"** can be used interchangeably with the term **"idursulfase".**

In the context of the present disclosure, the terms **"HIR-Fab-IDS"** as well as **"rIDS-FAB-HI", "rHI-FAB-IDS", "rIDS-FAB-HIR"** and **"rHIR-FAB-IDS"** are synonymous and refer to a hybrid recombinant protein of iduronate-2-sulfatase, covalently linked to the Fab fragment of a monoclonal antibody to the human insulin receptor. As used herein, **"HIR-Fab-IDS"** means, but is not limited to, iduronate-2-sulfatase with a monoclonal antibody fragment to the human insulin receptor. In particular (non-limiting) embodiments of the present invention, HIR-Fab-IDS variants are represented by a first amino acid sequence selected from SEQ ID NO:2, 8, 9, 10 or 11, and a second amino acid sequence selected from SEQ ID NO:4, 5, 12, 13, 14 or 15. In a particular embodiment of the present invention, the HIR-Fab-IDS is represented by the first amino acid sequence of SEQ ID NO:2 and the second amino acid sequence of SEQ ID NO:4.

In the context of the present disclosure, **"HIR-Mab-IDS"** is a recombinant iduronate-2-sulfatase fusion protein covalently linked to a full-length monoclonal antibody to the human insulin receptor. In particular (non-limiting) embodiments of the present invention, variants of HIR-Mab-IDS are variants according to US patent document US8834874 B2, 16.09.2014. In a specific embodiment of the present invention, the HIR-Mab-IDS is the amino acid sequence of the product of ArmaGen Technologies Inc. according to project AGT-182.

In the present disclosure, the term **"α-L-iduronidase"** as well as **"iduronidase", "laronidase", "IDUA"** refers to an enzyme involved in the hydrolysis of glycosaminoglycans such as dermatan sulfate and heparan sulfate. As used herein, the term **"α-L-iduronidase"** may be used interchangeably with the term **"laronidase".** Deficiency (genetically determined deficiency) of iduronidase, which occurs in mucopolysaccharidosis type I, leads to the gradual accumulation of glycosaminoglycans - heparan sulfate and dermatan sulfate - in the cells and tissues of the body.

**"HIR-Fab-IDUA",** as well as **"rIDUA-FAB-HI", "rHI-FAB-IDUA", "rIDUA-FAB-HIR", "rHIR-FAB-IDS",** is a fusion recombinant protein α-L-iduronidase, covalently linked to the Fab fragment of a monoclonal antibody to the human insulin receptor. In particular (non-limiting) embodiments of the present invention, HIR-Fab-IDUA variants are represented by a first amino acid sequence selected from SEQ ID NO:2, 8, 9, 10 or 11, and a second amino acid sequence selected from SEQ ID NO:6, 7.

**HIR-Mab-IDUA** is a fusion recombinant α-L-iduronidase protein covalently linked to a full-length monoclonal antibody to the human insulin receptor.

Therapeutic enzymes that can be included in the compounds of the present invention may be in their native form, as well as in the form of fragments consisting of parts of enzymes, or analogues of enzymes in which a variation has occured selected from the group consisting of substitution, addition, deletions, modifications of certain amino acids and combinations thereof, without limitation, provided that they have the same enzymatic activity as the native forms of the corresponding therapeutic enzymes. In particular (non-limiting) embodiments of the invention, enzyme fragments that have the activity of native forms of the corresponding enzymes can be used. Enzyme analogues are, without limitation, those enzymes that have different glycosylation characteristics and degrees of glycosylation due to expression of the known enzyme in different hosts, as well as varying degrees of substitution of specific amino acid residues of the corresponding enzyme relative to the standard sequence, where the degree of substitution is not 100% substitution. In private (non-limiting) embodiments of the invention, analogues of α-L-iduronidase, known from patents US5932211 A, 08/03/1999, US6153188 A, 11/28/2006 and US6541254 B1, 04/01/2003, as well as analogues of iduronate-2-sulfatase, can be used. An average person skilled in the art will understand that other fragments and analogues of the enzymes α-L-iduronidase and iduronate-2-sulfatase may be used, having the activity of native forms of the corresponding enzymes that are currently known in the art or will later become known.

Enzymes can be produced by methods conventional in the art, for example, through genetic recombination in animal cells, E. coli, yeast, insects, plants and living animals, etc., using various expression vectors well known to a person skilled in the art. The production methods are not limited to these and also include other methods for producing enzymes known to a person skilled in the art. For non-limiting examples of such methods for producing enzymes in the cells of various organisms, as well as non-limiting examples of expression vectors, see Sambrook et al., "Molecular Cloning: A Laboratory Manual" - CSH Press, Cold Spring Harbor, 1989, "Current Protocols in Molecular Biology , John Wiley & Sons, New York, 2001. In preferred embodiments of the invention, the enzymes are produced in mammalian cells. In the most preferred embodiments of the invention, the enzymes are produced in Chinese hamster ovary cells.

In certain preferred embodiments of the invention, the enzymes may be commercially available enzymes. In addition, enzymes may include an amino acid sequence that has homology of at least 80%, more particularly 90%, and even more particularly 91%, 92%, 93%, 94%, 95%, 96%, 97%, 98% or 99% or higher with the above enzymes or analogues thereof, and the enzymes may be obtained from microorganisms using recombinant technology or may be purchased from commercial sources, without limitation.

In the present disclosure, the term **"homology"** means the degree of similarity to the amino acid sequence of a wild-type protein or a nucleotide sequence encoding amino acids, and covers sequences that have the above degree of sequence similarity, expressed as a percentage, to the amino acid sequences or nucleotide sequences of the present invention. Homology can be determined by comparing two given sequences with the unaided eye or can be determined using a bioinformatic algorithm that allows homology analysis by aligning the sequences in question for comparison. The homology between two given amino acid sequences can be indicated as a percentage. There are effective automated algorithms that can be used in the GAP, BESTFIT, FASTA and TFASTA software modules of the Wisconsin Genetics software package (Genetics Computer Group, Madison, WI, USA). The alignment algorithm automated in these modules includes the sequence alignment algorithms of Needleman and Wunsch, Pearson and Lipman, and Smith and Waterman. Other algorithms that can be used for sequence alignment and homology determination are automated in the programs FASTP, BLAST, BLAST2, PSIBLAST and CLUSTAL W. Amino acid sequences and nucleotide sequences encoding enzymes and their analogs can be obtained from a well-known database such as, but not limited to, GenBank NCBI.

In some embodiments, the compound transport element comprises an IgG1 immunoglobulin Fab fragment consisting of a first amino acid sequence of at least 50, 55, 60, 65, 70, 75, 80, 81, 82, 83, 84, 85, 86 , 87, 88, 89, 90, 91, 92, 93, 94, 95, 96, 97, 98, 99% or more identical to SEQ ID NO:2.

In specific embodiments, the compound transport element comprises an IgG1 immunoglobulin Fab fragment consisting of a first amino acid sequence that is at least 80% or more identical to SEQ ID NO:2.

In some embodiments, a compound represented by a first amino acid sequence of at least 50, 55, 60, 65, 70, 75, 80, 81, 82, 83, 84, 85, 86, 87, 88, 89, 90, 91, 92, 93, 94, 95, 96, 97, 98, 99% or more identical to SEQ ID NO:2, and a second amino acid sequence at least identical to SEQ ID NO:4, used for the treatment or prevention of lysosomal enzyme deficiency in a subject with mucopolysaccharidosis type II.

In specific embodiments, a compound having a first amino acid sequence at least 80% or more identical to SEQ ID NO:2, and a second amino acid sequence at least identical to SEQ ID NO:4, used for the treatment or prevention of lysosomal enzyme deficiency in a subject with mucopolysaccharidosis type II.

The term **"amino acid"** refers to a group of carboxy-$\alpha$-amino acids that either occure in nature, i.e. which may be encoded by a nucleic acid directly or in the form of a precursor, or do not occure in nature. Individual naturally occurring amino acids are encoded by nucleic acids consisting of three nucleotides-called codons or base triplets. Each amino acid is encoded by at least one codon.

The term **"amino acid"** as used herein refers to naturally occurring carboxy-$\alpha$-amino acids including the following: alanine (three letter code: Ala, one letter code: A), arginine (Arg, R), asparagine (Asn, N), aspartic acid (Asp, D), cysteine (Cys, C), glutamine (Gln, Q), glutamic acid (Glu, E), glycine (Gly, G), histidine (His, H) , isoleucine (Ile, I), leucine (Leu, L), lysine (Lys, K), methionine (Met, M), phenylalanine (Phe, F), proline (Pro, P), serine (Ser, S), threonine (Thr, T), tryptophan (Trp, W), tyrosine (Tyr, Y) and valine (Val, V). Examples of amino acids that do not occur in nature (non-proteinogenic amino acids) include, Aad (alpha-aminoadipic acid), Abu (aminobutyric acid), Ach (alpha-aminocyclohexane carboxylic acid), Acp (alpha-aminocyclopentane carboxylic acid), Acpc ( 1-aminocyclopropane-1-carboxylic acid), Aib (alpha-aminoisobutyric acid), Aic (2-aminoindan-2-carboxylic acid; also called 2-2-Aic), 1-1-Aic (1-aminoindan-1-carboxylic acid), (2-aminoindan-2-carboxylic acid), allylglycine (allylGly), alloisoleucine (allo-Ile), Asu (alpha-aminosuberic acid, 2-aminooctanedioic acid), Bip (4-phenyl-phenylalanine-carboxylic acid), BnHP ((2S,4R)-4-hydroxyproline), Cha (beta-cyclohexylalanine), Cit (citrulline), cyclohexylglycine (Chg), cyclopentylalanine, beta-cyclopropylalanine, Dab (1,4-diaminobutyric acid), Dap ( 1,3-diaminopropionic acid, p-(3,3-diphenylalanine-carboxylic acid), 3,3-diphenylalanine, di-n-propylglycine (Dpg), 2-furylalanine, homocyclohexylalanine (HoCha), homocitrulline (HoCit), homocycloleucine, homoleucine (HoLeu), homoarginine (HoArg), homoserine (HoSer), hydroxyproline, Lys(Ac), (1) Nal (1-naphthylalanine), (2) Nal (2-naphthylalanine), 4-MeO-Ars (1-amino-4-(4-methoxyphenyl)-cyclohexane-1-carboxylic acid), nor-leucine (Nle), Nva (norvaline), omatine, 3-Pal (alpha-amino-3-pyridylalanine-carboxylic acid), 4-Pal (alpha-amino-4-pyridylalanine-carboxylic acid), 3,4,5,F3-Phe (3,4,5-trifluorophenylalanine), 2,3,4,5,6,F5-Phe (2,3,4,5,6-pentafluorophenylalanine), Pqa (4-oxo-6-(1-piperazinyl)-3(4H)-quinazoline-acetic acid (CAS 889958-08-1)), pyridylalanine , quinolylalanine, sarcosine (Sar), thiazolylalanine, thienylalanine, Tic (alpha-amino-1,2,3,4-tetrahydroisoquinoline-3-carboxylic acid), Tic(OH), Tie (tert-butylglycine) and Tyr(Me) , but are not limited to them.

The term **"amino acid sequence"** refers to polypeptides having amino acid sequences that differ to some extent from the polypeptide with the native sequence of the corresponding therapeutic enzyme. Typically, the amino acid sequence variants will have at least about 70% sequence identity to a polypeptide with the native sequence of the corresponding therapeutic enzyme. In one embodiment, the variant has about 80% or more sequence identity to a polypeptide with the native sequence of the corresponding therapeutic enzyme. In one embodiment, the variant has approximately 90% or more sequence identity to a polypeptide with the native sequence of the corresponding therapeutic enzyme. In one embodiment, the variant has about 95% or more sequence identity to a polypeptide with the native sequence of the corresponding therapeutic enzyme. In one embodiment, the variant has approximately 98% or more sequence identity to a polypeptide with the native sequence of the corresponding therapeutic enzyme. Amino acid sequence variants have substitutions, deletions and/or insertions at specific positions within the amino acid sequence of the native amino acid sequence of the corresponding therapeutic enzyme. Amino acids are designated by traditional names, one-letter and three-letter codes.

The term "first amino acid sequence" as used in this invention refers to the amino acid sequence of an IgG immunoglobulin in general, and to the light chain of an IgG immunoglobulin in particular. In specific, non-limiting embodiments, the first amino acid sequence is an IgG1 immunoglobulin amino acid sequence, an IgG1 immunoglobulin light chain amino acid sequence, a fragment of an IgG1 immunoglobulin light chain amino acid sequence selected from SEQ ID NO: 2, 8, 9, 10 or 11. In a particular embodiment the first amino acid sequence is the amino acid sequence of the immunoglobulin IgG light chain - SEQ ID NO:2.

The term "second amino acid sequence" as used in this invention refers to the amino acid sequence of an IgG immunoglobulin in general; to an IgG immunoglobulin heavy chain, to an IgG immunoglobulin heavy chain fragment, to an IgG immunoglobulin heavy chain fragment coupled to an enzyme sequence directly or by a linker, in particular. In specific (non-limiting) embodiments, the second amino acid sequence is an immunoglobulin IgG1 amino acid sequence, a heavy chain fragment amino acid sequence - SEQ ID NO:3, is an IgG1 immunoglobulin heavy chain fragment coupled to an iduronate-2-sulfatase sequence selected from SEQ ID NO :4, 5, 12, 13, 14 or 15.

The term **"transport element"** as used in this invention refers to a substance that is capable of carrying, transporting, delivering a therapeutic enzyme to the lysosomes of cells of various tissues. In particular, but not limited to, the transport element will specifically interact with an epitope, antigen, receptor or target in such a way as to ensure effective delivery to the lysosomes of neural tissue cells. Such an epitope, antigen, receptor or target in the context of the present disclosure may be the human insulin receptor or a part thereof, through which the delivery of a drug, peptide or protein is carried out, including through the BBB.

**"Immunoglobulin"** is a tetrameric molecule, the notion **"full-length antibody"** as used herein. In naturally occurring immunoglobulins, each tetramer consists of two identical pairs of polypeptide chains, with each pair having one "light" chain (approximately 25 kDa) and one "heavy" chain (approximately 50-70 kDa). The N-terminal portion of each chain includes a variable region of about 100 to 110 or more amino acids, primarily responsible for antigen recognition. The carboxyl portion of each chain defines a constant region primarily responsible for effector function.

In the context of the present disclosure, the term **"antibody"** is used in its broadest sense and includes, but is not limited to, monoclonal antibodies, polyclonal antibodies, multispecific antibodies (e.g., bispecific antibodies) formed from at least two intact antibodies, and antibody fragments provided that they have the required biological activity.

As used herein, **"antibody fragments"** contain a portion of an intact antibody that retains the ability to bind to an antigen. Examples of antibody fragments are Fab, Fab', F(ab')2 and Fv fragments; dimeric antibodies (diabody); linear antibodies; single chain antibody molecules, such as single chain Fab, scFv and multispecific antibodies formed from antibody fragments. The cleavage of antibodies by papain produces two identical antigen-binding fragments, called "Fab fragments", each having one antigen-binding site, and a residual "Fc-fragment", which name reflects its ability to crystallize readily.

**"Fab fragment", "Fab", "FAB"** is a monovalent fragment that contains heavy and light chain variable domains and also contains a light chain constant domain and a first heavy chain constant domain. As used herein, the transport element comprises the amino acid sequence of a Fab fragment of an IgG immunoglobulin, where the IgG immunoglobulin is IgG1, IgG2 or IgG4. In particular (non-limiting) embodiments of the present invention, the IgG immunoglobulin is IgG1. In a specific embodiment, the transport element comprises an amino acid sequence of an IgG1 immunoglobulin Fab fragment consisting of a first amino acid sequence selected from SEQ ID NO:2, 8, 9, 10 or 11, and a second amino acid sequence of SEQ ID NO:3. In a more specific embodiment, the transport element comprises the amino acid sequence of an IgG1 immunoglobulin Fab fragment consisting of SEQ ID NO:2 and SEQ ID NO:3.

As used herein, the term **"linker"** refers to a chemical linker or single-chain peptide linker that couples the therapeutic enzyme and the transport element of the compound of the present invention. The linker couples, for example, the monovalent linking element containing the CH2-CH3 domain of Ig and sFab, the target of which is the insulin receptor, i.e. a linker couples sFab to the C-terminus of the CH3-CH2 domain of Ig.

In some embodiments, the linker is a chemical linker. Single chain peptide linkers containing from one to twenty amino acids linked by peptide bonds can be used. In specific embodiments, the amino acids are selected from twenty naturally occurring (proteinogenic) amino acids. In other specific embodiments, one or more amino acids are selected from glycine, alanine, proline, asparagine, glutamine and lysine. In particular (non-limiting) embodiments of the present invention, one or more amino acids are selected from glycine, serine and leucine.

In specific embodiments of the invention, said linker is a single chain peptide whose amino acid sequence consists of at least 1 amino acid, preferably 1-2 amino acids. In particular (non-limiting) embodiments of the present invention, the amino acid sequence of the linker may consist of more than 1-2 amino acids, for example, 3 or 15 amino acids.

The coupling of the therapeutic enzyme and the transport element can be accomplished directly or using a variety of chemical linkers known in the art. In certain preferred (non-limiting) embodiments of the invention, coupling of the therapeutic enzyme and transport element can be accomplished using a variety of bifunctional protein coupling agents such as N-succinimidyl-3-(2-pyridyldithio)propionate (SPDP), succinimidyl-4-(N-maleimidomethyl)cyclohexane-1-carboxylate (SMCC), iminothiolane (IT), bifunctional imidoesters (such as dimethyl adipimidate·HCl), active esters (such as disuccinimidyl suberate), aldehydes (such as glutaraldehyde), bisazido compounds (such such as bis(p-azidobenzoyl)hexanediamine), bisdiazonium derivatives (such as bis(p-diazoniumbenzoyl)ethylenediamine), diisocyanates (such as toluene-2,6-diisocyanate) and bis-active fluorine compounds (such as 1,5-difluoro -2,4-dinitrobenzene). A person skilled in the art will also understand that other chemical and peptide linkers known in the art that are not expressly mentioned herein may be used for the purposes of the present invention.

In certain preferred embodiments of the invention, the linker may be a "cleavable linker" that facilitates the release of the therapeutic enzyme after transport of the compound into cells and tissues. For example, an acid-labile linker, a peptidase-sensitive linker, a photolabile linker, a dimethyl linker, or a disulfide-containing linker can be used (Chari R. et al., Cancer Res. 52, 1992, pp. 127-131; US 5,208,020, Chari Ravi J. et al., 05/04/1993). One skilled in the art will also appreciate that other cleavable linkers known in the art to facilitate the release of the therapeutic enzyme once the compound has entered the cells and tissues of the central nervous system may be used for the purposes of the present invention.

The term **"epitope"** is the region of an antigen that is bound by an antigen-binding protein, including an antibody. Epitopes can be defined as structural or functional. Functional epitopes are generally a subset of structural epitopes and have those residues that directly contribute to the affinity of the interaction. Epitopes can also be conformational, which are composed of non-linear amino acids, in other words, conformational epitopes are composed of non-sequential amino acids. Epitopes may include determinants, which are chemically active surface moieties of molecules, such as amino acids, sugar side chains, phosphoryl groups or sulfonyl groups, and they may have specific three-dimensional structural characteristics and/or specific charge characteristics. As used herein, the epitope is an insulin receptor epitope represented by SEQ ID NO: 1 described in Zhang B, Roth RA. (1991) Proc Natl Acad Sci U S A.; 88(21):9858-9862 and S A Prigent, K K Stanley, and K Siddle. (1990) J. Biol. 1991.

The **"insulin receptor"** (HIR) is a transmembrane glycoprotein (with a molecular weight of approximately 320,000 Da), which consists of two $\alpha$ subunits and two $\beta$ subunits linked by disulfide bridges (the $\alpha$ subunits are located extracellularly and contain an insulin-binding domain) and is involved in the regulation absorption and distribution of glucose, as well as the synthesis and accumulation of fats, proteins and carbohydrates in the human body. Insulin receptors and their extracellular insulin-binding domain (ECD) are widely known in the art, both structurally and functionally. The localization of insulin receptors most often refers to the cell surfaces of insulin-sensitive tissues, such as connective tissue cells, skeletal muscles, adipose tissue cells, liver cells, etc. See, for example, Yip et al. (2003), J Biol. Chem. 278 (30): 27329-27332; and Whittaker et al. (2005), J Biol Chem, 280 (22): 20932-20936. In one embodiment, the HIR herein is a human insulin receptor comprising the amino acid sequence set forth in Kasuya et al. (Biochemistry 32 (1993) 13531-13536).

The insulin receptor is expressed almost everywhere, and the use of such a delivery route can significantly increase the bioavailability of the recombinant enzyme and increase the effectiveness of therapy. Delivery to brain tissue is carried out through transcytosis through the capillary endothelium of the central nervous system, through interaction with the human insulin receptor.

In peripheral and brain tissues (Hawkes C. et al., 2004) expressing M6PR, internalization of the chimeric molecule can occur in both ways: antibody-HIR interaction and enzyme-M6PR interaction. In this case, targeted delivery to lysosomes occurs through interaction with M6P receptors. Internalization via HIR (human insulin receptor) results in entry into early order endosomes and subsequent transcytosis. In addition to delivering a functional enzyme to the CNS, many lysosomal storage diseases require improved internalization by certain peripheral tissues (diaphragm muscles in Pompe disease, liver and spleen in Hunter disease, kidneys in Fabry disease, etc.).

The term **"specific"** means that the molecule related to the term can form a complex with a specific region of another molecule. Binding can be detected by *in vitro* assays such as plasmon resonance (BIAcore, GE-Healthcare, Uppsala, Sweden). The specific interaction of a molecule with the binding site of another molecule (affinity of complex formation) is determined by the indicators $k_a$ (rate constant for the association of compounds to form a complex), $k_D$ (dissociation constant, dissociation of the complex) and $K_D(k_D/k_a)$. Binding or specific binding means a binding affinity $(K_D)$ of about $10^{-7}$ M or less, in one embodiment from about $10^{-8}$ M to about $10^{-13}$ M, in one embodiment from about $10^{-9}$ M to about $10^{-13}$ M.

The term "lysosome" refers to a cellular organelle - a type of vesicles that contains a number of enzymes (acid hydrolases) capable of breaking down macromolecules either from the cell itself (for example, when structural components of the cell are processed) or captured from the outside. Inherited defects or deficiencies of lysosomal enzymes (or other lysosomal components) can lead to the accumulation of undegraded metabolites. Glycosaminoglycans (formerly called mucopolysaccharides) are common polysaccharides of cell surfaces and extracellular matrix and structures. Enzyme deficiencies that interfere with the breakdown of glycosaminoglycan cause accumulation of glycosaminoglycan fragments in lysosomes and cause widespread changes in bone, soft tissue, and the central nervous system.

The **"activity"** of the enzyme(s) of the invention can be measured using any suitable test. Typically, pH determination and temperature determination can be adapted to the enzyme in question. Examples of tested pH values are pH 2, 3, 4, 5, 6, 7, 8, 9, 10, 11 or 12. Examples of tested temperatures are 30, 35, 37, 40, 45, 50, 55, 60, 65, 70, 80, 90 or 95°C. Preferred pH and temperature values are in the physiological range, such as pH values of 4, 5, 6, 7 or 8 and temperatures of 30, 35, 37 or 40°C. For example, protease activity can be measured using any test that uses a substrate that includes peptide bonds relevant to the specificity of the protease in question.

Examples of suitable enzyme tests are included in the experimental section, in particular see Example 2. As used herein, the term **"activity"** means **"enzyme activity", "specific activity", "enzyme specific activity"; "specific potency"** depending on the context of the description.

The term **"blood-brain barrier"** or **"BBB"** refers to the physiological barrier between the peripheral bloodstream and the brain and spinal cord, which is formed by tight junctions in the endothelial plasma membranes of brain capillaries, creating a tight barrier that limits the transport of molecules into the brain, even very small molecules such as urea (60 Da). The BBB in the brain, the blood-spinal cord barrier in the spinal cord, and the blood-retinal barrier in the retina are continuous capillary barriers in the CNS and are generally referred to herein as the blood-brain barrier (hereinafter also referred to as the BBB). The BBB also includes a barrier between the blood and the cerebrospinal fluid.

**"Pharmaceutical composition"** refers to a mixture of one or more of the compounds described herein, or physiologically/pharmaceutically acceptable salts or prodrugs thereof, with other chemical components, such as physiologically/pharmaceutically acceptable carriers and excipients. The purpose of the pharmaceutical composition is to facilitate the administration of the compound into the body.

The term **"effective amount"** of a compound, for example, in a pharmaceutical composition, refers to an amount that is effective in doses and for periods of time necessary to achieve the desired therapeutic or prophylactic result (effect) in a subject being treated, while reasonably observing benfit/risk.

The term **"pharmaceutically acceptable carrier"** refers to an ingredient of a pharmaceutical composition, other than the active substance (compound, agent, etc.), that is non-toxic to a subject. A pharmaceutically acceptable carrier includes, but is not limited to, a buffer, excipient, stabilizer or preservative. In particular (non-limiting) embodiments of the present invention, a pharmaceutically acceptable carrier is administered together with the compound

Pharmaceutical compositions that contain the compounds used according to the present invention are prepared for storage by mixing with optional pharmaceutically acceptable carriers, excipients or stabilizers (Remington's Pharmaceutical Sciences, 16th ed., ed. Osol A., 1980), preferably in the form of lyophilized compositions or aqueous solutions. Acceptable carriers, excipients or stabilizers are non-toxic to subjects at the dosages and concentrations employed and include buffers such as phosphate, citrate and other organic acid buffers; antioxidants including ascorbic acid and methionine; preservatives (such as octadecyldimethylbenzylammonium chloride; hexamethonium chloride; benzalkonium chloride; benzethonium chloride; phenol; butyl or benzyl alcohol; alkylparabens such as methyl or propylparaben; catechol; resorcinol; cyclohexanol; 3-pentanol and meta-cresol); low molecular weight polypeptides (containing less than about 10 residues); proteins such as serum albumin, gelatin or immunoglobulins; hydrophilic polymers such as poly(vinylpyrrolidone); amino acids such as glycine, glutamine, asparagine, histidine, arginine or lysine; monosaccharides, disaccharides and other carbohydrates, including glucose, mannose or dextrins; chelating agents such as EDTA; sugars such as sucrose, mannitol, trehalose or sorbitol; salt-forming counterions such as sodium; metal-containing complexes (eg Zn-protein complexes); and/or nonionic surfactants such as TWEEN™, PLURONICS™ or polyethylene glycol (PEG).

The pharmaceutical compositions used herein may also optionally contain more than one active substance (drug) active against lysosomal storage disease, optionally substances with additional activities that do not adversely affect each other. The type and effective amounts of such drugs depend, for example, on the amount of the therapeutic enzyme and transport element containing compound present in the composition and the clinical parameters of the subjects.

Administration of doses of the claimed therapeutic compound can be carried out by any suitable route well known to one skilled in the art, for example, by injection, such as intravenous, intramuscular or subcutaneous injection. The choice of the specific route of administration of a therapeutic compound is made by a person skilled in the art and depends, in particular, on whether the administration is short-term or long-term. One skilled in the art will appreciate that other routes of administration known in the art may be used to administer dosages of the claimed therapeutic compound, such as (without limitation), intrathecal administration, intra-arterial administration, intraperitoneal administration, transdermal administration, inhalation administration, buccal administration, intranasal administration, oral administration, sublingual administration or transnasal administration (Felice B.R., Wright T.L., Boyd R.B. Safety Evaluation of Chronic Intrathecal Administration of Idursulfase-IT in Cynomolgus Monkeys. - Toxicol Pathol. 2011 Aug;39(5):879-9, WO 2011/044542 A1).

In the context of the present disclosure, various dosing schedules can be considered, including, but not limited to, single or multiple administrations at different times, bolus administration, and pulsatile infusion.

**"Subject", "individual"** or **"patient"** is a mammal. Mammals include, but are not limited to, domestic animals (e.g., cows, sheep, cats, dogs, and horses), primates (e.g., humans and primates such as monkeys, particularly great apes), rabbits, and rodents (e.g., mice and rats). In some embodiments, the subject or patient is a human.

As used herein, the term **"treatment"** (and its grammatical variations such as "to treat" or "the process of treatment") refers to a clinical intervention with the purpose of changing the natural history of a disease in the individual being treated, and may be carried out either for prevention or in the process of development of clinical pathology. The desired treatment actions include, but are not limited to, prevention of onset or recurrence of the disease, relief of symptoms, reduction of any direct or indirect pathological consequences of the disease, prevention of metastases, reduction in the rate of disease progression, alleviation or temporary amelioration of the disease state, and remission or improvement of prognosis. In some embodiments, the compounds of the invention are used to delay the development of a disease or slow the progression of a disease.

The term **"CNS"** or **"central nervous system"** refers to the complex of nerve tissues that control body function and includes the brain and spinal cord.

As used herein, the term **"lysosomal storage disease"** (LSD; may also be referred to as **"lysosomal storage disorder"**) refers to a rare genetic disorder resulting in loss of lysosomal functions described above due to partial or complete loss of activity of one of the lysosomal enzymes. In this case, ERT is necessary to replenish an enzyme whose activity is completely or partially lost, or a missing enzyme. As used herein, the term **"lysosomal storage disease"** is used interchangeably with the term **"lysosomal storage disorders"** (also may be referred to as **"lysosomal storage disorder"**). Lysosomal storage diseases can be classified depending on the defect or deficiency of the enzyme into: (i) sphingolipidosis, (ii) mucopolysaccharidosis, (iii) glycogen storage disease, (iv) mucolipidosis, (y) oligosaccharidosis, (vi) lipidosis, (vii) disorder lysosomal transport and so on.

Below, lysosomal storage diseases will be described in more detail according to their classification.

As used herein, the term **"sphingolipidosis"** refers to a genetically determined deficiency syndrome of the lysosomal enzyme that hydrolyzes the carbohydrate side chains or choline side chains of sphingolipids. Diseases are classified according to the distribution of each stored lipid, such as Krabbe disease caused by galactocerebrosidase deficiency, Fabry disease caused by $\alpha$-galactosidase A deficiency, Niemann-Pick disease caused by sphingomyelinase deficiency, Gaucher disease caused by glucocerebrosidase deficiency, Tay-Sachs disease, caused by hexosaminidase A deficiency, etc., and they are the diseases that are inherited in an autosomal recessive manner, with the exception of Fabry disease, which is an X-linked genetic disease.

In the present disclosure, the term **"mucopolysaccharidosis"** (MPS) refers to a genetic deficiency syndrome of mucopolysaccharide hydrolase caused by deficiency of the carbohydrate chain degrading enzyme, sulfatase, acetyltransferase, etc. The main symptom of mucopolysaccharidosis (MPS) is excessive secretion of mucopolysaccharides in the urine. Currently, MPS is classified into 6 types, among which type I disease includes Hurler syndrome and Scheie syndrome, type II includes Hunter syndrome, type III includes Sanfilippo syndrome types A, B, C and D; Type IV includes Morquio syndrome A and B; Type VI includes Maroteaux-Lamy syndrome and type VII includes Sly syndrome.

As used herein, the term **"glycogen storage disease"** (also known as **"glycogenosis"**) refers to an inborn error of carbohydrate metabolism caused by glycogen storage and is divided into subtypes I-VII. The subtypes of glycogen storage disease associated with lysosomal storage disease (LSD) are types II (Pompe disease) and III b (Danon disease).

A detailed description of the lysosomal storage diseases described herein, as well as those disclosed in Table 1, is given in: The Online Metabolic & Molecular Bases of Inherited Diseases (Scriver's OMMBID), Part 16 (David L. Valle, Stylianos Antonarakis, Andrea Ballabio, Arthur L. Beaudet, Grant A. Mitchell, McGraw-Hill Education, 2007).

**"Mucopolysaccharidosis type I (MPS I)"** is an inherited metabolic disease caused by a defect in the enzyme $\alpha$-L-iduronidase (IDUA), whose function is to break down the acidic mucopolysaccharides heparan sulfate and dermatan sulfate. Insufficient levels of IDUA lead to pathological accumulation of these mucopolysaccharides in the tissues and organs of the patient, for example, in the heart, liver and central nervous system. Symptoms, including neurodegeneration and mental retardation, begin in childhood and, due to organ damage, early death can occur. A genetic deficiency in the carbohydrate breakdown enzyme $\alpha$-L-iduronidase causes a lysosomal storage disease known as mucopolysaccharidosis type I (MPS I). Severe MPS I is commonly known as Hurler syndrome, and is associated with a variety of problems such as mental retardation, corneal clouding, coarse facial features, heart disease, respiratory disease, enlarged liver and spleen, hernias, and joint stiffness. Patients suffering from Hurler syndrome usually die before the age of 10 years. In the moderate form, known as Hurler-Scheie syndrome, mental function is usually not greatly affected, but physical problems can also lead to death in adolescence or between the ages of twenty and twenty-nine. Scheie's syndrome is a mild form of MPS I. It is compatible with a normal life expectancy, but joint stiffness, corneal clouding and heart valve disease cause serious problems.

**"Mucopolysaccharidosis type II (MPS II)"** or "Hunter syndrome" is an X-linked inherited metabolic disorder caused by a deficiency of the enzyme iduronate 2-sulfatase (I2S). I2S is localized in lysosomes and plays an important role in the catabolism of glycosaminoglycans (GAGs) of heparan and dermatan sulfate. In the absence of the enzyme, these substrates accumulate in cells, eventually causing stagnation and then cell death and tissue destruction. Due to the widespread expression of the enzyme, different types of cells, organs and systems are affected in patients with MPS II. A characteristic clinical feature of this disease is degeneration of the central nervous system (CNS), which leads to cognitive impairment (eg, decreased IQ). In addition, MRI scans of patients revealed white matter damage, expansion of perivascular spaces in the brain parenchyma, ganglia, corpus callosum and brain stem, atrophy and ventriculomegaly (Wang et al. Molecular Genetics and Metabolism, 2009). The disease usually manifests itself in the first years of life with organomegaly and skeletal abnormalities. Some patients experience progressive loss of cognitive function, with most patients dying from disease-related complications in the first or second decade of life (Raluy-Callado M et al. Orphanet J Rare Dis. 2013;8:101;).

As used herein, the term **"neurological component"** refers to a disease or disorder that affects the central nervous system and/or the etiology of which is related to the central nervous system. Examples of CNS diseases or disorders include, but are not limited to, neuropathy, amyloidosis, cancer, ocular disease or disorder, viral or microbial infection, inflammation, ischemia, neurodegenerative disease, seizure disorder, behavioral disorders, and lysosomal storage disease. Specific examples of neurological disorders include, but are not limited to, neurodegenerative diseases (including, but not limited to, Lewy body disease, postmyelitis syndrome, Shy-Drager syndrome, olivopontocerebellar atrophy, Parkinson's disease, multiple system atrophy, striatonigral degeneration), tauopathies (including, but not limited to, Alzheimer's disease and supranuclear palsy), prion diseases (including, but not limited to, bovine spongiform encephalopathy, scrapie, Creutzfeldt-Jakob syndrome, kuru, Gerstmann-Strössler disease Schenker disease, chronic wasting disease, and fatal familial insomnia), palsy, motor neuron disease, and heterodegenerative disorders of the nervous system (including, but not limited to, Canavan disease, Huntington disease, neuronal ceroid lipofuscinosis, Alexander disease, Tourette syndrome, Menkes curly hair, Cockayne syndrome, Hallerwarden-Spatz syndrome, Lafora disease, Rett syndrome, hepatolenticular degeneration, Lesch-Nyhan syndrome and Unferricht-Lundborg syndrome), dementia (including, but not limited to, Pick's disease and spinocerebellar ataxia), cancer (e.g., cancer of the central nervous system and/or brain, including brain metastases from cancer in any area of the body).

The compounds of the invention can be used in therapy either individually or in combination with other agents. For example, a compound of the invention comprising a therapeutic enzyme and a transport element coupled by a linker may be co-administered with at least one additional therapeutic agent. In specific embodiments, the additional therapeutic agent is a therapeutic agent effective in treating the same neurological disorder for which the compound of the invention is used, or another neurological disorder. Examples of additional therapeutic agents include, but are not limited to, the various neurological drugs described above, including cholinesterase inhibitors (such as donepezil, galantamine, rovastigmine and tacrine), NMDA receptor antagonists (such as memantine), amyloid beta peptide aggregation inhibitors, antioxidants, $\gamma$-secretase modulators, nerve growth factor (NGF) mimics or NGF gene therapy agents, PPAR$\gamma$ agonists, HMS-CoA reductase inhibitors (statins), ampakines, calcium channel blockers, GABA receptor antagonists, glycogen synthase kinase inhibitors, immunoglobulin, intended for intravenous administration, muscarinic receptor agonists, nicotinic receptor modulators, active or passive immunization agents against amyloid beta peptide, phosphodiesterase inhibitors, serotonin receptor antagonists and antibodies to amyloid beta peptide. Such combination therapies as defined above include coadministration (where two or more therapeutic agents are included in the same or different compositions) and separate administration, in which case administration of a compound containing a therapeutic enzyme and a transport element coupled by a linker of the invention, can be performed before, simultaneously and/or after the administration of an additional therapeutic agent and/or adjuvant.

Some terms defined above may appear more than once, in which case each term should be defined independently of the other.

Hereinafter, exemplary embodiments of the present invention will be described in detail. Moreover, each of the explanations and implementations given as an example in this document may be valid for other explanations and implementations given as an example. Thus, all combinations of the various factors described herein are within the scope of the present invention. Moreover, the scope of the present invention is not limited to the specific description given below.

**Embodiments of the invention**

The present invention will now be described in more detail with reference to the following Examples. However, the Examples described below are for illustrative purposes only and are not intended to limit this invention.

**Example 1: Obtaining**

For obtaining the compounds, animal cells into which an animal cell expression vector was introduced were cultured and purified.

*Construction of Expression Vectors*

Amino acid sequences first LC-HIR-FAB-IDS (SEQ ID NO:2, 8, 9, 10 and 11) and second HC-HIR-FAB-IDS (SEQ ID NO:4, 5, 12, 13, 14, 15 )) to obtain HIR-FAB-IDS variants, including the signal peptide MDWTWRVFCLLAVAPGAHS, were converted into nucleotide sequences. For gene synthesis followed by cloning into the pCLN-1 vector, the following HindIII restriction site and Kozak sequence were added to the 5' end of the sequences. Two stop codons and an XbaI restriction site were added to the 3' end of the sequences.

Codon optimization of the nucleotide sequence for Chinese hamster cells was carried out using the resources: http://gcua.schoedl.de and http://www.kazusa.or.jp. The synthesis of the light and heavy chain LC-HIR-FAB-IDS and HC-HIR-FAB-IDS was carried out at GenArt (USA) and transferred as part of the vectors pRA1675 and pRA1673 (containing the genes LC-HIR-FAB-IDS and HC-HIR- FAB-IDS, respectively).

The sequences HC-HIR-FAB-IDS, LC-HIR-MAB from plasmids pRA1675 and pRA1673 were cloned into the expression vector pCLN-1 at HindIII/XbaI sites to obtain vectors pGNR-055-007 (pCLN-1-HC-HIR-FAB- IDS) and pGNR-055-008 (pCLN-1-LC-HIR-FAB-IDS). The resulting vectors were linearized at BspHI/PvuI sites.

The vectors of the present invention were constructed in accordance with molecular biology techniques well known in the art. See Brown T, "Gene Cloning" (Chapman & Hall, London, GB, 1995); Watson R, et al., "Recombinant DNA", 2nd Ed. (Scientific American Books, New York, NY, US, 1992); Alberts B, et al., "Molecular Biology of the Cell" (Garland Publishing Inc., New York, NY, US, 2008); Innis M, et al., Eds., "PCR Protocols. A Guide to Methods and Applications" (Academic Press Inc., San Diego, CA, US, 1990); Erlich H, Ed., "PCR Technology. Principles and Applications for DNA Amplification" (Stockton Press, New York, NY, US, 1989); Sambrook J, et al., "Molecular Cloning. A Laboratory Manual" (Cold Spring Harbor Laboratory Press, Cold Spring Harbor, NY, US, 1989); Bishop T, et al., "Nucleic Acid and Protein Sequence. A Practical Approach" (IRL Press, Oxford, GB, 1987); Reznikoff W, Ed., "Maximizing Gene Expression" (Butterworths Publishers, Stoneham, MA, US, 1987); Davis L, et al., "Basic Methods in Molecular Biology" (Elsevier Science Publishing Co., New York, NY, US, 1986), Schleef M, Ed., "Plasmid for Therapy and Vaccination" (Wiley-VCH Verlag GmbH, Weinheim, DE, 2001).

*Obtaining a monoclonal cell line*

Parental cell line CHO-S. Cells were cultured at 37°C, 5% CO2, 70% humidity, in BalanCD CHO Growth A medium (Invitrogen). Stable transfection was carried out on a NEON device (Invitrogen) according to the standard protocol for CHO cells using two linearized plasmids pCLN-1-HC-HIR-FAB-IDS and pCLN-1-LC-HIR-FAB-IDS. An equimolar DNA ratio was used for transfection.

After 48 hours, the transfected pools were seeded into minipools in 96-well plates in BalanCD CHO Growth A medium containing 600 $\mu$g/ml of the selective antibiotic neomycin. The minipools were cultivated under stationary conditions for 10 days at 37°C, 5% CO2, 70% humidity, after which a series of productivity screenings were carried out using EIA. The leader minipool was cloned into semi-solid ClonaCell Flex medium (STEMCELL) for single colony growth using 6-well plates. The plates were incubated for 10 days at 37°C, 5% CO2, 70% humidity.

During the screening, the concentration of the target protein HIR-FAB-IDS secreted into the culture liquid was determined. The determination was carried out by the sandwich EIA method, using instead of primary antibodies a fragment of the recombinant human insulin receptor (5 $\mu$g/ml in a carbonate-bicarbonate buffer pH 9.6); horseradish peroxidase-conjugated rat antibodies to iduronate-2-sulfatase (diluted 1:10000) were used as second antibodies. Developed with a solution of tetramethylbenzidine, the reaction was stopped with 0.5 M sulfuric acid.

Based on the results of screening of 6-well plates, 20 leading minipools with productivity from 0.4 to 2.1 mg/l were selected, which were transferred to flasks for adaptation to suspension cultivation. To adapt to suspension cultivation, 3 passages were carried out, after which 20 minipools were frozen.

Clones were selected automatically using a ClonePix robot (Molecular Devices) into 96-well plates. The resulting clones were screened. For this purpose, a 7-day cultivation process was simulated in batch mode, in which the clones were assessed according to the following parameters: the dynamics of culture viability, the dynamics of viable cell density, the concentration of the target HIR-FAB-IDS, and the dependence of volumetric production on the cumulative cell density.

*Cultivation of producer cells*

Cultivation of producer cells expressing HIR-FAB-IDS was carried out in a fed-batch mode using the BalanCD CHO Growth A culture medium (Irvine Scientific) and the nutrient additive BalanCD CHO Feed 2 (Irvine Scientific) for 12 days in bioreactor with an overhead stirrer at a temperature of 37°C and pH 6.9. After the cultivation process, the culture liquid was clarified by depth filtration and transferred for isolation.

*Isolation and purification*

Isolation and purification are carried out using standard technologies for protein isolation and purification. The compound can be isolated or purified by any method known in the art to isolate or purify protein, for example by chromatography (eg, ion exchange, high performance liquid chromatography, affinity assisted, protein A and size-sorting column chromatography), centrifugation, differential solubility, or any other standard protein isolation or purification technique.

**Example 2. Determination of the level of enzymatic activity of the Fab fragment of an antibody to the insulin receptor coupled to the amino acid sequence of iduronate-2-sulfatase**

The specific activity of HIR-FAB-IDS and HIR-MAB-IDS is determined fluorimetrically using 4-mutilumbelliferyl-L-iduronide-2-sulfate (4-MUS) (Moscerdam Substrates, the Netherlands) (Voznyi YV et al., 2001; Tolun AA, et al., 2012; 9. Johnson BA et al., 2013; Azadeh M et al., 2017). During the analysis, the substrate is hydrolyzed by iduronate-2-sulfatase to produce 4-mutylumbelliferyl-L-iduronide (MUBI), which is hydrolyzed by iduronidase (IDUA, Aldurazyme, Genzyme, USA) to 4-methylumbelliferyl (4-MU), which is detected fluorimetrically, using the following fluorimeter settings: fluorescence excitation at a wavelength of 450 nm and fluorescence detection at a wavelength of 365 nm. The calibration curve is constructed using a standard solution 4-MU (Sigma-Aldrich, USA). During the analysis, the mixture is first incubated at 37 °C, pH = 4.5 for 4 hours, then IDS is added in an amount of 12 $\mu$g and the mixture is further incubated at 37 °C for 24 hours. The incubation is stopped by adding 0.2 ml 0.5 M sodium carbonate pH=10.3. HIR-FAB-IDS exhibits specific enzymatic (iduronate-2-sulfatase) activity towards the substrate 4-MU-$\alpha$IdoA-2S (4-methylumbeliferyl $\alpha$-L-idopyranosiduronate-2-sulfate).

*Instruments and equipment*

1. Thermostatic shaker PST-60 HL-4 (BioSan, Latvia or similar).
2. Multifunctional reader Spectra Max M3 (Molecular Devices, USA or similar).
3. Software for multifunctional reader Soft Max Pro (Molecular Devices, USA or similar).
4. Analytical balance ML 204 (Mettler Toledo, Switzerland or similar quality).
5. 96-wellblack plate with non-absorbent surface (Corning, USA, cat. No. 3916 or similar).

*Reagents*

1. 4-methylumbelliferone sodium salt (Sigma-Aldrich, Product No. M1508 or equivalent).
2. Recombinant human $\alpha$-L-iduronidase (R&D SYSTEM, Product No. 4119-GH or equivalent quality).
3. 4-methylumbelliferyl $\alpha$-L-idopyranosiduronic acid 2-sulfate sodium salt, 0.5 mg/vial (USBiological, Product No. 017551 or equivalent).
4. Sodium acetate anhydrous (AppliChem Panreac, Product No. 141633.1211 or equivalent quality).
5. Acetic acid (Fluka, Product No. 49199 or similar quality).
6. Bovine serum albumin (Sigma, Product No. A7030 or equivalent quality).
7. Sodium carbonate (Sigma-Aldrich, Product No. S7795 or equivalent quality).
8. Sodium bicarbonate (Sigma-Aldrich, Product No. S6297 or equivalent quality).
9. Lead diacetate trihydrate (Aldrich, Product No. 467863 or equivalent quality).
10. Sodium phosphate dihydrate (Sigma-Aldrich, Product No. 71643 or equivalent quality).
11. Anhydrous citric acid (AppliChem Panreac, Product No. 141808 or equivalent quality).

*Preparation of solutions*

Sample dilution solution, pH (5.5 $\pm$ 0.2). About 4.1 g of anhydrous sodium acetate and 0.5 g of bovine serum albumin are placed in a 1-liter beaker and dissolved in 700 ml of purified water. pH of the solution is adjusted to a value of (5.5 $\pm$ 0.2) with acetic acid. The resulting solution is transferred to a 1-liter volumetric flask, the volume of the solution is adjusted to the mark with purified water, mixed and filtered through a membrane filter with a pore diameter of 0.45 $\mu$m.

Storage life is 3 months at temperatures from 2 to 8 °C.

Substrate dilution solution, pH (5.00 $\pm$ 0.05). 4.1 g of anhydrous sodium acetate, 1.9 g of lead diacetate trihydrate sre placed in a beaker with a volume capacity of 500 ml, 450 ml of purified water is added, the mixture is stirred until dissolved. The pH of the solution is adjustered to a value of (5.00 ± 0.05) with acetic acid (about 1.1 ml). The resulting solution is transferred to a 500 ml volumetric flask, the volume of the solution is adjusted to the mark with purified water, mixed and filtered through a membrane filter with a pore diameter of 0.45 μm.

Storage life is 3 months at temperatures from 2 to 8 °C.

Substrate solution (1.25 mmol/l). 0.83 ml of the substrate dilution solution is added to a bottle containing the substrate (4-methylumbelliferyl α-L-idopyranosiduronic acid 2-sulfate sodium salt), and gently mixed. The resulting solution is divided into 0.2 ml aliquots and frozen.

Storage life is 3 months at a temperature not exceeding minus 70 °C.

Solution of recombinant human α-L-iduronidase. 400 μl of purified water is added to a bottle containing recombinant human α-L-iduronidase (10 μg) and mixed. The resulting solution is divided into 0.1 ml aliquots and frozen.

Storage life is 3 months at a temperature not exceeding minus 70 °C.

0.1 M solution of citric acid. About 19.2 g of anhydrous citric acid is placed in a 1-liter capacity flask, dissolved in 900 ml of purified water, the volume of the solution is adjusted to the mark with purified water, and filtered through a 0.22-μm membrane filter.

Storage life is 3 months at temperatures from 15 to 25 °C.

0.2 M solution of sodium phosphate disubstituted. About 35.6 g of sodium phosphate dihydrate is placed in a 1-liter capacity flask, dissolved in 900 ml of purified water, the volume of the solution is adjusted to the mark with purified water, and filtered through a 0.22-μm membrane filter.

Storage life is 3 months at temperatures from 15 to 25 °C.

Phosphate-citrate buffer solution, pH (4.5 ± 0.1). 55.0 ml of a 0.1 M citric acid solution and 45.0 ml of a 0.2 M dibasic sodium phosphate solution are mixed in a 100 ml capacity measuring flask and filtered through a membrane filter with a pore diameter of 0.22 μm.

Storage life is 3 months at temperatures from 15 to 25 °C.

0.5 M sodium bicarbonate solution. About 42.0 g of sodium bicarbonate is placed in a 1-liter measuring flask, dissolved in 900 ml of purified water, the volume of the solution is adjusted to the mark with purified water and mixed and filtered through a membrane filter with a pore diameter of 0.22 μm.

Storage life is 6 months at temperatures from 15 to 25 °C.

0.5 M sodium carbonate solution. About 53.0 g of sodium carbonate is placed in a 1-liter capacity measuring flask, dissolved in 900 ml of purified water, the volume of the solution is adjusted to the mark with purified water and mixed and filtered through a membrane filter with a pore diameter of 0.22 μm.

Storage life is 6 months at temperatures from 15 to 25 °C.

Stop solution, pH (10.8 ± 0.5). 900 ml of 0.5 M sodium carbonate solution and 100 ml of 0.5 M sodium bicarbonate solution are added into a 1-liter capacity measuring flask, mixed and filtered through a membrane filter with a pore diameter of 0.22 μm.

Storage life is 6 months at temperatures from 15 to 25 °C.

Primary solution of 4-methylumbelliferone sodium salt (100 μmol/ml). About 1.0 g (exactly weighed) of 4-methylumbelliferone sodium salt is placed in a 50 ml capacity measuring flask, 30 ml of purified water is added, mixed, and adjusted to the mark with purified water. The solution is divided into 2.0 μl aliquots.

Storage life is 6 months at a temperature not exceeding minus 18 °C.

Standard solution of 4-methylumbelliferone sodium salt (50 nmol/ml).

An aliquot of the primary solution of 4-methylumbelliferone sodium salt is kept in a water bath for 5 minutes at a temperature of 37 °C. Serial dilutions are prepared according to the following scheme:

| Solution No. | Starting p-p 4-MU[à] | Solution No. 2 | Stop solution | Concentration, nmol/ml |
|---|---|---|---|---|
| 1 | 100 | - | 9900 | 1000 |
| 2 | - | 500 | 9500 | 50 |
| [à] 4-MU - 4-methylumbelliferone sodium salt. | | | | |

Dilutions of the standard solution of 4-methylumbelliferone sodium salt. Dilutions of a working standard solution of 4-methylumbeliferone sodium salt are prepared according to the following scheme:

| Nos. of dilutions | Volume of standard solution (50 nmol/ml), ml | Volume of stop solution, ml | Concentration, nmol/ml |
|---|---|---|---|
| 1 | 0 | 5.00 | 0.0 |

(continued)

| Nos. of dilutions | Volume of standard solution (50 nmol/ml), ml | Volume of stop solution, ml | Concentration, nmol/ml |
|---|---|---|---|
| 2 | 0.05 | 4.95 | 0.5 |
| 3 | 0.10 | 4.90 | 1.0 |
| 4 | 0.15 | 4.85 | 1.5 |
| 5 | 0.20 | 4.80 | 2.0 |
| 6 | 0.25 | 4.75 | 2.5 |
| 7 | 0.30 | 4.70 | 3.0 |
| 8 | 0.35 | 4.65 | 3.5 |
| 9 | 0.40 | 4.60 | 4.0 |

Dilutions of the test sample. The test sample is diluted with sample dilution solution to a concentration of 1 mg/ml based on the actual HIR-FAB-IDS content stated on the certificate. Serial dilutions are then prepared according to the following scheme:

| No. of tube | HIR-FAB-IDS concentration, ng/ml | Volume of solution, $\mu$l | | | | | | | | |
|---|---|---|---|---|---|---|---|---|---|---|
| | | HIR-FAB-IDS solution (1 mg/ml) | No. of tube | | | | | | | Sample dilution solution |
| | | | 1 | 2 | 3 | 4 | 5 | 6 | 7 | |
| 1 | 100000 | 100 | - | - | - | - | - | - | - | 900 |
| 2 | 10000 | | 100 | - | - | - | - | - | - | 900 |
| 3 | 1000 | | | 100 | - | - | - | - | - | 900 |
| 4 | 100 | | | | 100 | - | - | - | - | 900 |
| 5 | 10 | | | | | 100 | - | - | - | 900 |
| 6 | 5 | | | | | | 100 | - | - | 100 |
| 7 | 2,5 | | | | | | | 100 | - | 100 |
| All dilutions are stored on ice until testing begins. | | | | | | | | | | |

In further studies, solutions from test tubes No. 5-7 are used.

*Carrying out analysis*

1st enzymatic reaction

10 $\mu$l of each dilution of the test sample (in 2 replicates) are added to the wells of the plate; the sample dilution solution is used as a reference solution. 20 $\mu$l of substrate solution (4-methylumbelliferyl $\alpha$-L-idopyranosiduronic acid 2-sulfate sodium salt) is added. The plate is covered with film and incubation in a thermostatic shaker for 4 hours at a temperature of (37.0 $\pm$ 0.2) °C and a stirring speed of 250 rpm is performed. The plate should be protected from light during the entire incubation period.

2nd enzymatic reaction

At the end of incubation, 20 $\mu$l of phosphate-citrate buffer solution is added to the wells of the plate to stop the first enzymatic reaction. Then 10 $\mu$l of recombinant $\alpha$-L-iduronidase solution is added to each well and mixed gently. The plate is covered with film and incubation in a thermostatic shaker for 22-26 hours at a temperature of 37 °C and a stirring speed of 250 rpm is performed. The plate should be protected from light during the entire incubation period.
To stop the reaction, 200 $\mu$l of stop solution is added into each well with the incubated mixture.
Dilutions of the standard solution of 4-methylumbelliferone sodium salt in amounts of 260 $\mu$l are added into the empty wells of the plate.

The fluorescence signal is measured in the wells of the plate at an excitation wavelength of 365 nm and a detection wavelength of 460 nm.

Evaluation of results

Based on the results of measuring the fluorescence signal in the wells with dilutions of a standard solution of 4-methylumbelliferone sodium salt, a linear relationship between the fluorescence signal and the concentration of 4-methylumbelliferone sodium salt (nmol/ml) is plotted. Using the linear function equation, the concentration of 4-methylumbelliferone produced during the reaction in wells with dilutions of test samples and a reference solution (nmol/ml) is calculated.

Specific activity A, in units/$\mu$g, for each dilution of test samples is calculated using the formula:

$$A = 0{,}26 \times \frac{(m - m_0) \times 1000}{240 \times C \times 0{,}01}$$

where: m is the concentration of 4-methylumbeliferone accumulated during the reaction in wells with a given dilution of the test sample (average value of 2 replicates), nmol/ml;

$m_o$ is the concentration of 4-methylumbeliferone produced during the reaction in wells with a reference solution (average value of 2 replicates), nmol/ml;

0.26 is the final volume of dilutions of the standard solution and the test sample added to the wells of the plate to measure the fluorescence signal, ml;

240 - time of the 1st enzymatic reaction, min;

C is the content of HIR-Fab-IDS in each of the prepared dilutions of the test sample, ng/ml;

0.01 is the volume of dilutions of the test sample added to the 1st enzymatic reaction, ml;

1000 is the conversion factor from ng to $\mu$g.

The final specific activity value for the test sample is calculated as the average of the specific activity calculated for each dilution.

The results obtained for determining the enzymatic activity of the HIR-FAB-IDS and HIR-MAB-IDS variants in comparison with the drug Elapraza are presented in Table 2.

Table 2. Evaluation of iduronate-2-sulfatase activity of the HIR-FAB-IDS and HIR-MAB-IDS variants against the unmodified iduronate-2-sulfatase enzyme (Elaprase)

| Names of samples | Activity, U(nmol/min)/ $\mu$g |
|---|---|
| HIR-FAB-IDS (SEQ ID NO:2 and SEQ ID NO:4) | 27.0 |
| HIR-FAB-IDS (SEQ ID NO:2 and SEQ ID NO:5) | 20.0 |
| HIR-FAB-IDS (SEQ ID NO:8 and SEQ ID NO:12) | 25.2 |
| HIR-FAB-IDS (SEQ ID NO:9 and SEQ ID NO:13) | 30.2 |
| HIR-FAB-IDS (SEQ ID NO:10 and SEQ ID NO:14) | 27.5 |
| HIR-FAB-IDS (SEQ ID NO:11 and SEQ ID NO:15) | 23.4 |
| HIR-MAB-IDS | 11.0 |
| HIR-MAB-IDS (AGT-182) (the value is taken from patent for AGT-182 US8834874 B2, Fig.12 example 4, p. 35) | 0.85 (51.7+-7 nmol/hour/pg) US8834874 B2 |
| Elaprase (Shire Human Genetic Therapies Inc., USA) | 14.6 |

As it can be seen from the results presented in Table 2, the HIR-FAB-IDS drug exhibits specific enzymatic (iduronate-2-sulfatase) activity towards the substrate 4-MU-$\alpha$IdoA-2S (4-methylumbeliferyl $\alpha$-L-idopyranosiduronate-2-sulfate ) 27.0 U/$\mu$g. In this case, the free recombinant enzyme iduronate-2-sulfatase exhibits an activity of 14.6 U/$\mu$g. Full-length insulin receptor antibody coupled to the amino acid sequence of iduronate-2-sulfatase (HIR-MAB-IDS) exhibits lower specific activity (11.0 U/$\mu$g) compared to HIR-FAB-IDS.

As a result of the studies, it was shown that iduronate-2-sulfatase in the composition of HIR-FAB-IDS retains the main functional (enzymatic) properties at the level of the free recombinant enzyme. At the same time, the specific activity of HIR-FAB-IDS is higher than for the drug Elaprase and HIR-MAB-IDS (AGT-182)

Thus, an increase in the enzymatic activity of a compound containing a transport element, which is a Fab fragment of an IgG immunoglobulin, specific to an insulin receptor epitope, coupled directly or via a linker to a therapeutic enzyme, was detected, in comparison with a therapeutic enzyme without a transport element, and with a therapeutic enzymewith a transport element represented by MAB.

Example 3. Analysis of the interaction of modified Fab fragments of antibodies to the insulin receptor with the human and mouse insulin receptor.

To perform a quantitative assessment of the interaction with the insulin receptor of full-length recombinant monoclonal antibodies, variants of Fab fragments, as well as their modifications with IDS, an enzyme-linked immunosorbent assay (ELISA) technique, sandwich format, is used (Kim C, Seo J, Chung Y et al. , 2017). The method is based on the binding of proteins HIR-MAB (83-14 Mab), HIR-MAB-IDS and HIR-FAB-IDS with a protein fragment of the human insulin receptor (Human Insulin Receptor protein fragment (Abcam cat#ab200510)) or mouse insulin receptor ( mInsR, R&D Systems, Inc., Cat. No. 7544-MR).

The detection process is carried out after incubation of the complex of the human insulin receptor and the protein under study with secondary antibodies - horseradish peroxidase-conjugated Fab-specific goat antibodies to human immunoglobulin (Anti-Human IgG (Fab specific) - Peroxidase (Sigma-Aldrich, cat#A0293)).

Previously described mouse antibodies to the human insulin receptor (INSR/Insulin Receptor alpha Antibody 83-14 AHR0221 Life technologies, cat#AHR0221) and to the mouse insulin receptor (Mouse Anti-Human Insulin Receptor Clone 83-14 mAb Cell sciences) were used as a positive control, cat#MAI1). Detection is carried out using horseradish peroxidase-conjugated goat polyclonal antibodies to mouse immunoglobulin (Goat pAb to Ms IgG (HRP) Abcam, cat# ab97023).

The results obtained for determining the interaction of the tested molecules with the human and mouse insulin receptor are presented in Tables 3 and 4.

Table 3. Binding of modified Fab fragments of antibodies to the insulin receptor with the human insulin receptor (Abcam cat#ab200510).

| No. | Name of the sample | EC50, ng/ml | Receptor binding from antibody 83-14 mAb, % |
|---|---|---|---|
| 1 | INSR/Insulin Receptor alpha Antibody 83-14 (Life technologies, cat#AHR0221) | 32.15 | 102 |
| 2 | Mouse Anti-Human Insulin Receptor Clone 83-14 mAb (Cell sciences, cat# MAII) | 33.10 | 100 |
| 3 | HIR-FAB-IDS (SEQ ID NO:2 and SEQ ID NO:4) | 24.31 | 136 |
| 4 | HIR-FAB-IDS (SEQ ID NO:2 and SEQ ID NO:5) | 50.25 | 66 |
| 5 | HIR-FAB-IDS (SEQ ID NO:8 and SEQ ID NO:12) | 120.55 | 27 |
| 6 | HIR-FAB-IDS (SEQ ID NO:9 and SEQ ID NO:13) | 60.35 | 55 |
| 7 | HIR-FAB-IDS (SEQ ID NO:10 and SEQ ID NO:14) | 35.10 | 94 |
| 8 | HIR-FAB-IDS (SEQ ID NO:11 and SEQ ID NO:15) | 155.67 | 21 |
| 9 | HIR-MAB-IDS | 26.19 | 126 |

Table 4. Binding of modified Fab fragments of insulin receptor antibodies to the mouse insulin receptor (R&D Systems, Inc., cat. no. 7544-MR).

| No. | Name of the sample | EC50, ng/ml | Receptor binding, % |
|---|---|---|---|
| 1 | INSR/Insulin Receptor alpha Antibody 83-14 | > 800 | 0 |
| 2 | Mouse Anti-Human Insulin Receptor Clone 83-14 mAb | > 800 | 0 |
| 3 | HIR-FAB-IDS (SEQ ID NO:2 and SEQ ID NO:4) | > 800 | 0 |
| 4 | HIR-FAB-IDS (SEQ ID NO:2 and SEQ ID NO:5) | > 800 | 0 |
| 5 | HIR-FAB-IDS (SEQ ID NO:8 and SEQ ID NO:12) | > 800 | 0 |
| 6 | HIR-FAB-IDS (SEQ ID NO:9 and SEQ ID NO:13) | > 800 | 0 |
| 7 | HIR-FAB-IDS (SEQ ID NO:10 and SEQ ID NO:14) | > 800 | 0 |
| 8 | HIR-FAB-IDS (SEQ ID NO:11 and SEQ ID NO:15) | > 800 | 0 |
| 9 | HIR-MAB-IDS | > 800 | 0 |

Thus, it was demonstrated that HIR-FAB-IDS specifically interacts with the extracellular domain of the alpha subunit of the human insulin receptor, but does not interact with the extracellular domain of the mouse insulin receptor. The binding of Fab fragments of antibodies to the insulin receptor with the human insulin receptor is comparable to the control antibody (83-14 mAb) and exceeds the binding to the similar full-length antibody to the insulin receptor (HIR-MAB-IDS).

The interaction of HIR-FAB-IDS with the human insulin receptor is fundamental to ensure active transcytosis of the molecule to cell lysosomes, in general, through the BBB, in particular.

**Example 4. Analysis of tissue distribution of radiolabeled Fab fragments of an antibody to the insulin receptor coupled to the amino acid sequence of iduronate-2-sulfatase [125I]-HIR-Fab-IDS** (SEQ ID NO:2 and SEQ ID NO:4), **[ 125I]-HIR-Mab-IDS, [125I]-IDS (control) after intravenous administration in cynomolgus monkeys.**

The experimental animals were injected once into the left femoral vein with the corresponding molecule of the test substance at a target dose of 2 ml/kg body weight by intravenous bolus injection over 1-2 minutes. The target radioactive dose level was 1 MBq/kg animal body weight for all molecules studied.

The animal receiving the test substance was sedated by intramuscular injection of ketamine hydrochloride before intravenous administration of an excess dose of Doletil (sodium pentaborbitone). Animals were humanely euthanized 2 hours after administration of the test substance. During sedation, 2 ml of blood was collected from each animal from the cephalic vein and centrifuged to obtain plasma. The concentration of radioactivity was measured in the blood plasma of animals. Once death was confirmed, each animal was quickly frozen in a mixture of solid carbon dioxide in hexane. Once completely frozen, the bodies were placed in a mold containing 2% (w/v) aqueous carboxymethylcellulose paste. Several longitudinal sagittal sections (nominally 30 $\mu$m) were taken at at least 5 body levels and, if necessary, 3 head levels for each animal.

Sections attached to Filmolux 610 tape (Neschan) were lyophilized in a GVD03 benchtop freeze dryer (Girovac Ltd) and placed on FUJI X-ray plates (type BAS-MS, Raytek Scientific Ltd). Blood standards labeled with the isotope [125I] with the appropriate activity, also prepared in the form of sections with a nominal thickness of 30 $\mu$m, were placed on radiographic plates.

After developing in a copper-coated lead box for 7 days, the radiographic plates were processed using a FUJI FLA-5000 radiographic system (Raytek Scientific Ltd). Electronic images were analyzed using a PC-based image analysis system (Seescan 2 software, LabLogic Ltd). [125I] standards included in each autoradiogram were used to construct calibration curves over a range of radioactivity concentrations.

Tissue concentration data were recorded as ng equivalents of [125I]HIR-Fab-IDS [125I]HIR-Mab-IDS and [1251]

-IDS (control).

The results were expressed to three significant numbers with no more than two decimal places. Data tables were computer generated and individual data were rounded appropriately.

[$^{125}$I]HIR-Fab-IDS, [$^{125}$I]HIR-Mab-IDS and [$^{125}$I]-IDS (control) were administered to male cynomolgus monkeys as a single intravenous dose at nominal dose levels of 0.0020 mg/kg, 0.0015 mg/kg and 0.0010 mg/kg body weight. Euthanasia of the animal was performed 2 hours after the administration of each drug, after which the animals were frozen for studies using whole-body autoradiography.

After intravenous administration, the drugs were absorbed and widely distributed throughout the tissues of the body, and at the time of killing, all tissues studied were exposed to it. The lower limit of quantification was 0.2, 0.03, and 0.097 ng drug equivalent/g for all animal tissue concentration measurements, respectively.

Radioactivity concentrations in plasma and blood of the animal treated with [$^{125}$I]HIR-Fab-IDS were 2.55 and 3.11 ng eq/g, respectively (blood:plasma ratio 1.22). Quantifiable levels of radioactivity were present in several brain regions, including the medulla oblongata (1.09 ng eq/g), cerebral cortex (1.03 ng eq/g), cerebellum (0.908 ng eq/g), hypothalamus (0.869 ng eq/g) /g), arborescent substance of the cerebellum (0.799 ng eq/g), pons (0.793 ng eq/g) and medulla (0.562 ng eq/g); the TP ratio ranged from 0.22 to 0.43

Radioactivity concentrations in plasma and blood of the animal treated with [$^{125}$I]HIR-Mab-IDS were 2.13 and 1.86 ng eq/g, respectively (blood:plasma ratio 0.87). Quantifiable levels of radioactivity were present in a number of areas of the animal's brain. These areas included the medulla oblongata (0.803 ng eq/g), cerebellar arboresum (0.606 ng eq/g), cerebellum (0.494 ng eq/g), cerebral cortex (0.453 ng eq/g), pons (0.438 ng eq/g). d), medulla (0.288 ng eq/g) and hypothalamus (0.279 ng eq/g), TP concentration ratios ranged from 0.13 to 0.38.

Plasma and blood concentrations in the [$^{125}$I]IDS-treated animal were 0.910 and 0.753 ng eq/g, respectively (blood:plasma ratio 0.83). Quantifiable levels of radioactivity were present throughout the brain (0.113 ng eq/g), but concentrations were below the limit of quantification (0.097 ng eq/g) in all regions examined: medulla oblongata, cerebral cortex, cerebellum, hypothalamus, cerebellar arboresum, pons and medulla. Concentrations in CNS tissues were significantly lower than in plasma and blood.

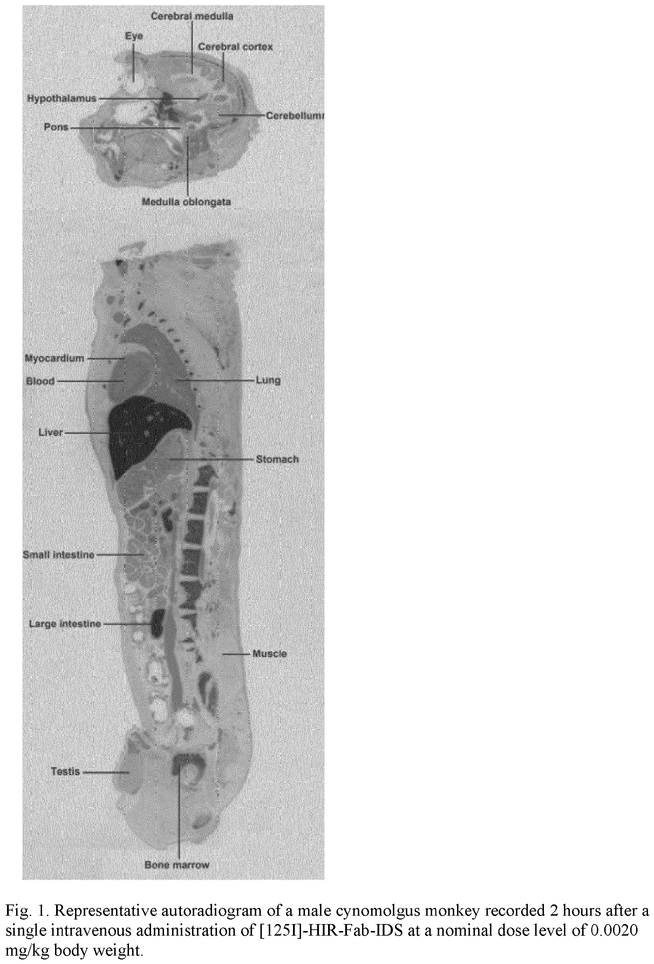

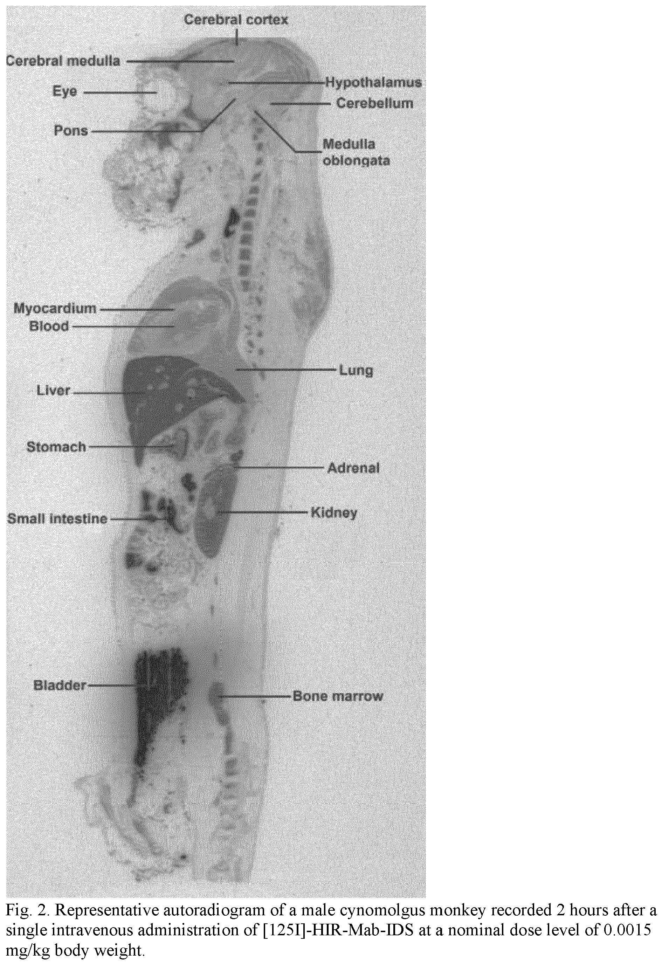

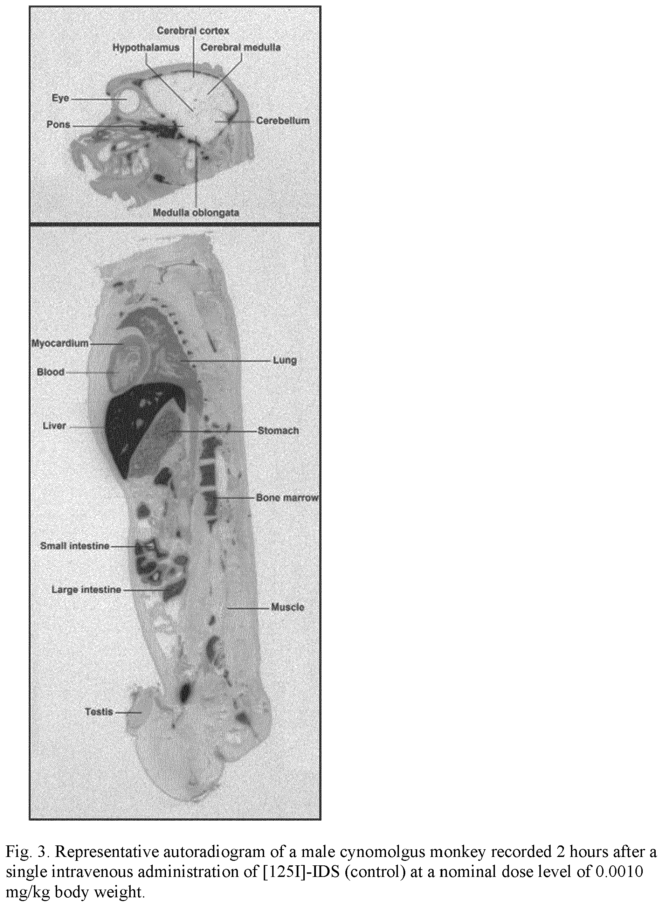

These results evidence that HIR-Fab-IDS-bound substances, as well as HIR-Mab-IDS-bound substances, compared with IDS-bound substances, penetrated the blood-brain barrier, CNS tissues were exposed to the test substance or its labeled metabolites, and radioactivity transmitted through the blood had little effect on the determination of concentration in CNS tissues (Figures 1 and 2). Moreover, HIR-Fab-IDS showed a wider distribution in body tissue compared to the HIR-Mab-IDS molecule (Table 5, Figure 3).

It should be noted that in example 10 of invention US8834874 B2 (AGT-182 or HIR-MAB-IDS), the penetration efficiency into the human brain is estimated at 1% of the administered dose per 1000 grams of brain tissue based on experimental data on the penetration of AGT-182 (HIR - MAB-IDS) into the brain in rhesus monkeys. It is stated that at this level of penetration, it is expected to achieve 20% of iduronate-2-sulfatase (IDS) activity in the human brain, which will eliminate the accumulation of glycosaminoglycans in the brain of a sick person.

Taking into account the data obtained on penetration into the brain of cynomolgus monkeys (Table 5), we can conclude that the present compound penetrates into the brain of cynomolgus monkeys almost 2 times better than AGT-182 (HIR-MAB-IDS), so - 0 .84 ng eq/g HIR-FAB-IDS versus 0.43 ng eq/g for HIR-MAB-IDS. This accordingly means that HIR-FAB-IDS penetrates the human brain with 2% efficiency. Based on the calculations shown in Example 10 of US8834874B2, 40% of the iduronate-2-sulfatase activity in human brain is expected to be achieved, excluding the increased activity of HIR-FAB-IDS compared to HIR-MAB-IDS (AGT-182), cm Table 2.

If we take into account the fact of the increase in specific activity of HIR-FAB-IDS over HIR-MAB-IDS (AGT-182), shown in example 2, table 2 - 27 U/$\mu$g for the present compound and 11 U/$\mu$g for the opposite one, as well as greater penetration into the brain: 27/11 = 2.4 times higher specific iduronate-2-sulfatase activity, 0.84/0.43 = 1.95 times greater penetration into the brain, which results in a 20% restoration of IDS activity in the patient's brain during therapy HIR-MAB-IDS (AGT-182) and 20% * 2.4 * 1.95 = 93.6% restoration of iduronate-2-sulfatase activity in the brain of a patient with MPS type II from the level of IDS activity in the brain of a healthy person. Thus, the present invention will almost completely restore the enzymatic function of IDS in the brain of a sick person, while the opposed AGT-182 (HIR-MAB-IDS) only restores by 20% (93% vs 20%).

Thus, it has been found that the administration of a compound containing a transport element, which is a Fab fragment of an IgG immunoglobulin specific for an insulin receptor epitope, coupled directly or by a linker to a therapeutic enzyme, provides a higher degree of replacement of the activity of the therapeutic enzyme in the human brain in comparison with compounds containing Mab.

Table 5. Radioactivity concentrations in cynomolgus monkey tissues after a single intravenous administration of [$^{125}$I] -HIR-Fab-IDS at a nominal dose level of 0.0020 mg/kg body weight

| Tissue | Radioactivity concentrations in tissues | | |
|---|---|---|---|
| | [$^{125}$I] HIR-Fab-IDS (SEQ ID NO:2 и SEQ ID NO:4) | [$^{125}$I] HIR-Mab-IDS (AGT-182) | [$^{125}$I]-IDS |
| Plasma | 2.55 | 2.13 | 0.91 |
| Blood | 3.11 | 1.86 | 0.75 |
| Adrenal cortex | 18.3 | 5.29 | 4.47 |
| Adrenal medulla | 11.5 | 2.17 | 3.26 |
| Aortic wall | 3.29 | 1.96 | BLQ |
| Bone marrow | 6.37 | 3.26 | 4.39 |
| Periosteum | 3.32 | BLQ | 0.95 |
| Brown adipose tissue | 2.51 | 0.97 | 0.54 |
| Bulbourethral gland | 0.98 | BLQ | 0.60 |
| Mucous membrane of the blind intestine | 1.72 | 0.19 | 0.25 |
| Epididymis | 3.09 | 0.31 | 0.53 |
| Renal cortex | 10.1 | 4.25 | 4.33 |
| Renal medulla | 6.37 | 2.39 | 1.70 |
| Mucous membrane of the large intestine | 1.42 | 0.41 | 0.24 |
| Liver | 21,4 | 7.63 | 18.0 |
| Lung | 4.15 | 1.10 | 2.35 |
| Muscles | 0.51 | 0.09 | 0.28 |
| Myocardium | 3.78 | 0.91 | 1.25 |
| Nasal mucosa | 1.18 | 0.61 | 0.72 |
| Esophageal wall | 1.53 | 0.51 | 0.48 |
| Pancreas | 2.68 | 0.70 | 0.69 |
| Mucous membrane of the straight intestine | 1.15 | 0.39 | 0.27 |
| Salivary glands | 1.01 | 0.23 | 0.82 |
| Seminal gland | 2.08 | BLQ | BLQ |
| Derma | 1.20 | 0.20 | 0.39 |
| Mucous membrane of the small intestine | 3.14 | 0.62 | 1.62 |
| Spinal cord | 0.57 | 0.41 | 0.17 |
| Spleen | 17.9 | 10.4 | 11.6 |
| Testics | 1.34 | 0.18 | 0.41 |
| Tongue | 0.92 | 0.30 | 0.64 |
| Trachea | 0.65 | 0.32 | 0.45 |
| Bladder wall | 7.30 | 3.49 | 4.18 |

(continued)

| Tissue | Radioactivity concentrations in tissues | | |
|---|---|---|---|
| Brain (entire) | 0.84 | 0.43 | 0.11 |
| Cerebral cortex | 1.03 | 0.45 | BLQ |
| Cerebral Medulla | 0.56 | 0.29 | BLQ |
| Cerebellum | 0.91 | 0.49 | BLQ |
| Hypothalamus | 0.87 | 0.28 | BLQ |
| Medulla oblongata | 1.09 | 0.80 | BLQ |
| Pons | 0.79 | 0.44 | BLQ |
| Arborescent substance of the cerebellum | 0.80 | 0.61 | BLQ |
| Epiphyse | 1.03 | 0.39 | BLQ |
| Hypophysis | 0.80 | 0.60 | BLQ |
| NA - Not applicable BLQ - Concentration below the lower limit of quantitation | | | |

The present description presents compounds containing therapeutic enzymes that exhibit their specific enzymatic activity in these compounds, and transport elements capable of interacting with the insulin receptor, while having the ability to transport therapeutic enzymes to tissue lysosomes, including through the BBB to lysosomes of nervous tissue. Thus, the compounds exhibit high activity and improved ability to be transported to lysosomes of tissue cells of various organs, including lysosomes of nervous tissue cells, which suggests the use of the present invention for enzyme replacement therapy for the treatment or prevention of a subject with a lysosomal storage disease.

**Claims**

1. A compound containing a therapeutic enzyme and a transport element that are coupled to one another directly or by a linker, wherein said transport element is a Fab fragment of immunoglobulin IgG specific to an insulin receptor epitope.

2. The compound of claim 1, containing the therapeutic enzyme and the transport element that are coupled to one another directly or by a linker, wherein said transport element is a Fab fragment of immunoglobulin IgG specific to an insulin receptor epitope, and is capable of transporting said therapeutic enzyme through a blood-brain barrier.

3. The compound of claim 2, wherein the insulin receptor epitope is represented by amino acid sequence SEQ ID NO: 1.

4. The compound of claim 3, wherein the therapeutic enzyme and the transport element are coupled by a linker.

5. The compound of claim 4, wherein said linker is a peptide linker containing one or more amino acids.

6. The compound of claim 5, wherein said linker is a peptide linker containing one or more amino acids selected from glycine, serine and leucine

7. The compound of claim 1, wherein the transport element contains an amino acid sequence of a Fab fragment of immunoglobulin IgG, wherein the immunoglobulin IgG is IgG1, IgG2 or IgG4.

8. The compound of claim 1, wherein the transport element contains an amino acid sequence of a Fab fragment of immunoglobulin IgG, wherein the immunoglobulin IgG is IgG1.

9. The compound of claim 8, wherein the transport element contains a Fab fragment of immunoglobulin IgG1, consisting of a first amino acid sequence that is at least 80% identical to SEQ ID NO:2 and a second amino acid sequence SEQ ID NO:3.

10. The compound of claim 8, wherein the transport element contains a Fab fragment of immunoglobulin IgG1, consisting of a first amino acid sequence selected from SEQ ID NO:2, 8, 9, 10 or 11, and a second amino acid sequence SEQ ID NO:3.

11. The compound of claim 8, wherein the transport element contains a Fab fragment of immunoglobulin IgG1, consisting of a first amino acid sequence SEQ ID NO:2 and a second amino acid sequence SEQ ID NO:3.

12. The compound of claim 1, containing a therapeutic enzyme for treatment or prevention of a lysosomal enzyme deficiency in a lysosomal storage disease.

13. The compound of claim 1, containing a therapeutic enzyme for treatment or prevention of a lysosomal enzyme deficiency in a lysosomal storage disease with a neurological component.

14. The compound of claim 1, containing a therapeutic enzyme for treatment or prevention of a lysosomal enzyme deficiency in a lysosomal storage disease with a neurological component, said therapeutic enzyme is selected from the group consisting of iduronate -2-sulfatases, $\alpha$-L-iduronidases.

15. The compound of claim 14, containing a therapeutic enzyme for treatment or prevention of a lysosomal enzyme deficiency in a lysosomal storage disease with a neurological component, said therapeutic enzyme is selected from the group consisting of iduronate -2-sulfatases, a fragment of iduronate-2-sulfatase having iduronate-2-sulfatase activity, or an iduronate-2-sulfatase analogue.

16. The compound of claim 14, containing a therapeutic enzyme for treatment or prevention of a lysosomal enzyme deficiency in a lysosomal storage disease with a neurological component, said therapeutic enzyme is selected from the group consisting of $\alpha$-L-iduronidases, a fragment of $\alpha$-L-iduronidase having $\alpha$-L-iduronidase activity, or an $\alpha$-L-iduronidase analogue.

17. The compound of claim 1, wherein said transport element is capable of transporting said enzyme to lysosomes.

18. The compound of claim 17, wherein said transport element is capable of transporting said enzyme to lysosomes of neural tissue cells.

19. The compound of claims 1-2, represented by a first amino acid sequence at least 80% identical to SEQ ID NO:2, and a second amino acid sequence at least 80% identical to SEQ ID NO:4, used for treatment or prevention of lysosomal enzyme deficiency in a subject having mucopolysaccharidosis type II.

20. The compound of claims 1-2, represented by a first amino acid sequence selected from SEQ ID NO:2, 8, 9, 10 or 11, and a second amino acid sequence selected from SEQ ID NO:4, 5, 12, 13, 14 or 15, used for treatment or prevention of lysosomal enzyme deficiency in a subject having mucopolysaccharidosis type II.

21. The compound of claim 19, represented by a first amino acid sequence of SEQ ID NO:2 and a second amino acid sequence of SEQ ID NO:4, used for treatment or prevention of lysosomal enzyme deficiency in a subject having mucopolysaccharidosis type II.

22. The compound of claim 19, represented by a first amino acid sequence of SEQ ID NO:2 and a second amino acid sequence of SEQ ID NO:4, used for treatment or prevention of lysosomal enzyme deficiency in a subject having mucopolysaccharidosis type II with a neurological component.

23. The compound of claim 18, represented by the a amino acid sequence of SEQ ID NO:2 and a second amino acid sequence of SEQ ID NO:5, used for treatment or prevention of lysosomal enzyme deficiency in a subject having mucopolysaccharidosis type II with a neurological component.

24. The compound of claims 1-2, represented by a first amino acid sequence SEQ ID NO:2, and a second amino acid sequence SEQ ID NO:6, used for treatment or prevention of lysosomal enzyme deficiency in a subject having mucopolysaccharidosis type I.

25. The compound of claim 22, represented by a first amino acid sequence SEQ ID NO:2, and a second amino acid sequence SEQ ID NO:6, used for treatment or prevention of lysosomal enzyme deficiency in a subject having mucopolysaccharidosis type I with a neurological component.

26. The compound of claim 23, represented by a first amino acid sequence SEQ ID NO:2, and a second amino acid sequence SEQ ID NO:7, used for treatment or prevention of lysosomal enzyme deficiency in a subject having mucopolysaccharidosis type I with a neurological component.

27. Use of the compound of claims 1-15, 17-22 for obtaining a pharmaceutical composition containing an effective amount of said compound and a pharmaceutically acceptable carrier.

28. Use of the compound of claims 1-15, 17-22 for treatment or prevention of a lysosomal enzyme deficiency in a subject having a lysosomal storage disease, wherein said use includes administering to said subject an effective amount of said compound.

29. The use according to claim 28, wherein the lysosomal storage disease is mucopolysaccharidosis.

30. The use according to claim 28, wherein the lysosomal storage disease is mucopolysaccharidosis type II with a neurological component.

31. The use according to claim 28, wherein the lysosomal storage disease is mucopolysaccharidosis type II with a neurological component.

32. The use according to claim 28, wherein the lysosomal storage disease is mucopolysaccharidosis type II.

Fig. 1. Representative autoradiogram of a male cynomolgus monkey recorded 2 hours after a single intravenous administration of [125I]-HIR-Fab-IDS at a nominal dose level of 0.0020 mg/kg body weight.

Fig. 2. Representative autoradiogram of a male cynomolgus monkey recorded 2 hours after a single intravenous administration of [125I]-HIR-Mab-IDS at a nominal dose level of 0.0015 mg/kg body weight.

Fig. 3. Representative autoradiogram of a male cynomolgus monkey recorded 2 hours after a single intravenous administration of [125I]-IDS (control) at a nominal dose level of 0.0010 mg/kg body weight.

## INTERNATIONAL SEARCH REPORT

| | International application No. |
| --- | --- |
| | PCT/RU 2022/050251 |

**A. CLASSIFICATION OF SUBJECT MATTER**
See supplemental sheet

According to International Patent Classification (IPC) or to both national classification and IPC

**B. FIELDS SEARCHED**

Minimum documentation searched (classification system followed by classification symbols)
C07K 19/00, A61K 47/66, 47/68, 38/46, 39/395, 48/00, A61P 43/00, C12N 9/96, 9/14

Documentation searched other than minimum documentation to the extent that such documents are included in the fields searched

Electronic data base consulted during the international search (name of data base and, where practicable, search terms used)
PatSearch (RUPTO Internal), USPTO, PAJ, Espacenet

**C. DOCUMENTS CONSIDERED TO BE RELEVANT**

| Category* | Citation of document, with indication, where appropriate, of the relevant passages | Relevant to claim No. |
| --- | --- | --- |
| X | RU 2673038 C2 (OOO "MEZHDUNARODNY BIOTEKHNOLOGICHESKY TSENTR "GENERIUM") 21.11.2018, the abstract, the claims | 1-32 |
| X | WO 2015/009961 A1 (ARMAGEN TECHNOLOGIES, INC.) 22.01.2015, the abstract, the claims | 1, 27-28 |
| A | WO 2011/044542 A1 (ARMAGEN TECHNOLOGIES, INC) 14.04.2011, the abstract, the claims | 1-32 |
| A | US 2014/0178350 A (BIOASIS TECHNOLOGIES, INC) 26.06.2014 | 1-32 |
| A | RU 2744593 C2 (ESTEV FARMASIUTIKALS, S. A. et al.) 11.03.2021, the abstract | 1-32 |
| A | RU 2010134412 A1 (BAIOMARIN FARMASIUTIKAL INK.) 27.02.2012 | 1-32 |

| ☐ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 09 November 2022 (09.11.2022) | 01 December 2022 (01.12.2022) |

| Name and mailing address of the ISA/ RU | Authorized officer |
| --- | --- |
| Facsimile No. | Telephone No. |

Form PCT/ISA/210 (second sheet) (April 2005)

INTERNATIONAL SEARCH REPORT

International application No.

PCT/RU 2022/050251

*C07K 19/00* (2006.01)
*C12N 9/96* (2006.01)
*A61K 47/66* (2017.01)
*A61K 47/68* (2017.01)
*A61K 38/46* (2006.01)
*A61K 39/395* (2006.01)
*A61K 48/00* (2006.01)
*C12N 9/14* (2006.01)

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8834874 B2
- US 5932211 A
- US 6153188 A
- US 6541254 B1
- US 5208020 A, Chari Ravi J.
- WO 2011044542 A1

### Non-patent literature cited in the description

- **RODNEY J.Y. HO.** Biotechnology and biopharmaceuticals: transforming proteins and genes into drugs. Wiley-Blackwell, 2013, 380
- **MUENZER J. et al.** *Genet Med,* August 2006, vol. 8 (8), 465-473
- **SAMBROOK et al.** Molecular Cloning: A Laboratory Manual. CSH Press, Cold Spring Harbor, 1989
- Current Protocols in Molecular Biology. John Wiley & Sons, 2001
- **CHARI R. et al.** *Cancer Res.,* 1992, vol. 52, 127-131
- **ZHANG B ; ROTH RA.** *Proc Natl Acad Sci U S A.,* 1991, vol. 88 (21), 9858-9862
- **S A PRIGENT ; K K STANLEY ; K SIDDLE.** *J. Biol.,* 1990, vol. 1991
- **YIP et al.** *J Biol. Chem.,* 2003, vol. 278 (30), 27329-27332
- **WHITTAKER et al.** *J Biol Chem,* 2005, vol. 280 (22), 20932-20936
- **KASUYA et al.** *Biochemistry,* 1993, vol. 32, 13531-13536
- Remington's Pharmaceutical Sciences. 1980
- **FELICE B.R. ; WRIGHT T.L. ; BOYD R.B.** Safety Evaluation of Chronic Intrathecal Administration of Idursulfase-IT in Cynomolgus Monkeys. *Toxicol Pathol.,* August 2011, vol. 39 (5), 879-9
- **DAVID L. VALLE ; STYLIANOS ANTONARAKIS ; ANDREA BALLABIO ; ARTHUR L. BEAUDET ; GRANT A ; MITCHELL.** The Online Metabolic & Molecular Bases of Inherited Diseases (Scriver's OMMBID). McGraw-Hill Education, 2007
- **RALUY-CALLADO M et al.** *Orphanet J Rare Dis.,* 2013, vol. 8, 101
- **BROWN T.** Gene Cloning. Chapman & Hall, 1995
- **WATSON R et al.** Recombinant DNA. Scientific American Books, 1992
- **ALBERTS B et al.** Molecular Biology of the Cell. Garland Publishing Inc, 2008
- PCR Protocols. A Guide to Methods and Applications. Academic Press Inc, 1990
- PCR Technology. Principles and Applications for DNA Amplification. Stockton Press, 1989
- **SAMBROOK J et al.** Molecular Cloning. A Laboratory Manual. Cold Spring Harbor Laboratory Press, Cold Spring Harbor, 1989
- **BISHOP T et al.** Nucleic Acid and Protein Sequence. A Practical Approach. IRL Press, 1987
- Maximizing Gene Expression. Butterworths Publishers, 1987
- **DAVIS L et al.** Basic Methods in Molecular Biology. Elsevier Science Publishing Co, 1986
- Plasmid for Therapy and Vaccination. Wiley-VCH Verlag GmbH, 2001